# EUROPEAN PATENT APPLICATION

(11) **EP 2 441 459 A1**
(43) Date of publication of application: **18.04.2012**
(21) Application number: 10730196.2
(22) Date of filing: 07.06.2010
(51) Int. Cl.: A61K 31/7076, A61P 25/00, A61P 25/28

(54) **5'-METHYLTHIOADENOSINE NEUROPROTECTIVE PROPERTIES**

(30) Priority: 11.06.2009 ES 200930298
(71) Applicant: Proyecto de Biomedicina Cima, S.L., 31008 Pamplona - Navarra (ES)
(72) Inventor: VILLOSLADA DÍAZ, Pablo, E-31008 Pamplona (ES)
(74) Representative: Ponti Sales, Adelaida
(86) International application number: PCT/ES2010/070374
(87) International publication number: WO 2010/142827

(57) **Abstract**

The present invention relates to the use of MTA, its pharmaceutically acceptable salts and/or prodrugs thereof as active ingredient in the manufacture of a medicament for the prevention or treatment of nerve cell death or damage, a neuroprotective medicament, a medicament for the regeneration of nerve cells, and a medicament for the prevention or treatment of a neurological or psychiatric disease. The present invention also relates to a method of prevention or treatment of nerve cell death or damage, a method of neuroprotection, a method of regenerating nerve cells and a method of prevention or treatment of a neurological or psychiatric disease.

## Description

### Field of the invention

This invention applies to the area of therapeutics for neurological, psychiatric disorders, and ageing. In particular, it relates to the neuroprotective effect of the small molecule 5'-methylthioadenosine.

### Background of the invention

Ageing, neurological, and psychiatric disorders cause death and damage to nerve cells. Frequent and relevant insults to the nervous system include neuronal degeneration, ischemia, inflammation, immune responses, trauma, and cancer, among other. As a consequence of these, nerve cells can die within minutes or hours or survive to this initial insult in an impaired state that activates neurodegeneration, ending equally in cellular death.

Given the importance of the nervous system in enabling basic motor skills and sensing, there exists an interest in finding therapeutic weapons to protect the nervous system.

Neuroprotection aims to the preservation, recovery, cure, or regeneration of the nervous system, its cells, structure, and function (Vajda et al. 2002, J Clin Neurosci 9:4-8). A goal of neuroprotection is to prevent or minimize the effects of an original insult to the nervous system, or to prevent or minimize the consequences of endogenous or exogenous noxious processes causing damage to axons, neurons, synapses, and dendrites.

Treatment strategies in general are frequently based on the modulation of a single proposed injury factor. Although such treatments can be shown to be beneficial in highly constrained animal models, they are less likely to prove efficacious in the more complex human disorder that involves more variable degrees of injury severity in a genetically diverse population (Faden and Stoica 2007, Arch Neurol.; 64:794-800). Importantly, since the presumed mechanisms of neurone death are both complex and varied, such as oxidative stress, mitochondrial dysfunction, protein aggregation, apoptosis, and inflammation (Youdim et al. 2005, TIPS 26:27-35), single compounds having multipotential effects on multiple injury mechanisms are desirable.

Several neuroprotective drugs are under investigation including the following classes: anti-inflammatory agents, N-methyl D-aspartate (NMDA) antagonists, α-amino-3-hydroxy-5-methyl-4-isoxazole propionic acid (AMPA) antagonists, dexanabinol, sodium channel blockers, thyrotropin-releasing hormone (TRH), growth factors, glucocorticoids, caffeinol, opioid antagonists, apoptosis inhibitors, free radical trappers/scavengers, erythropoietin, calcium channel blockers, magnesium sulfate, statins.

The ability of these pharmacological agents to limit secondary biochemical damage and cell death has been well established in numerous animal models of stroke, head injury, and spinal cord injury, yet the results of such neuroprotective treatment strategies in human injury have been disappointing (Faden and Stoica 2007, Arch Neurol.; 64:794-800). As such, there is an ongoing need for providing drugs with neuroprotective properties, which have preferably multipotential effects.

5'-methylthioadenosine (MTA) is a lipophilic sulfur-containing adenine nucleoside produced from S-adenosylmethionine (SAM), during the synthesis of the poliamines spermine, and spermidine. MTA is a suitable drug for oral formulations due to its small size and hydrophilic character.

MTA has been described to have immunomodulatory activity by suppressing the production of proinflammatory genes and cytokines (interferon-y, tumor necrosis factor-α, and inducible nitric oxide synthase) and increasing the production of antiinflammatory cytokines (interleukin-10); and reversing brain autoimmune disease; thereby suggesting benefits for multiple sclerosis and other autoimmune diseases (Moreno et al. Diss. Abstr. Int., C 2007, 68(1), 111; Ann Neurol 2006; 60: 323-334). In this regard, WO2006097547 discloses MTA for use in the prevention and/or treatment of autoimmune diseases, such as multiple sclerosis (MS), and in the prevention and/or treatment of transplant rejection. Futher, US 4,454,122 (Bioresearch S.r.l.) discloses the use of MTA as an anti-inflammatory, antipyretic, and analgesic compound.

In two studies by Kang et al. (Zhongguo Shenjing Kexue Zazhi 1999, 15(4), 289-296; Yike Daxue Xuebao 1999, 26(5), 318-320), it has been suggested that MTA could partially inhibit reactive astrogliosis. Astrogliosis (astrocytosis) is an abnormal increase in the number of astrocytes due to the destruction of nearby neurons, typically because of hypoglycemia or oxygen deprivation (hypoxia). Astrogliosis is a major pathological finding in Amyotropic Lateral Sclerosis (ALS).

EP 526866 (Bioresearch S.p.A.) discloses that adenosine compounds are suitable for therapeutic use in various ischemia situations, such as cases of cerebral or myocardial ischemia, or for ischemia of any type of organs or tissues.

In the review by Avila et al. (Int J Biochem Cell Biol. 2004; 36(11):2125-30) the authors suggest that MTA can effect cellular processes in many ways, influencing numerous critical responses of the cell including regulation of gene expression, proliferation, differentiation and apoptosis. They propose a therapeutic potential for MTA given the observations in models of liver damage and cancer.

In an exhaustive effort to explore the neuroprotective potential of MTA, it has been surprisingly found that MTA indeed is effective in protecting astrocytes and neurons from the cerebral cortex deprived from oxygen and glucose; in protecting *Cornu Ammonis* (CA1) hippocampal neurons from cell death after global ischemia; in protecting oligodendrocytes from excitotoxicity and optic nerves tissue damage after ischemia; in protecting a mixed glial-neuronal culture subjected to excitotoxicity; in decreasing the number of dystrophic axons and in preventing the myelin and axonal loss.

### Summary of the invention

The present invention relates to the use of MTA, its pharmaceutically acceptable salts and/or prodrugs thereof as active ingredient in the manufacture of a medicament for the prevention or treatment of nerve cell death or damage. In other words, the present invention relates to MTA, its pharmaceutically acceptable salts and/or prodrugs thereof for use in the prevention or treatment of nerve cell death or damage. Similarly, the present invention also relates to a method of prevention or treatment of nerve cell death or damage comprising administering to a subj ect in need thereof an effective amount of MTA, its pharmaceutically acceptable salts and/or prodrugs thereof.

The present invention also relates to the use of MTA, its pharmaceutically acceptable salts and/or prodrugs thereof as active ingredient in the manufacture of a neuroprotective medicament. In other words, the present invention also relates to MTA, its pharmaceutically acceptable salts and/or prodrugs thereof for use as a neuroprotective drug. Similarly, the present invention relates to a method of neuroprotection comprising administering to a subject in need thereof an effective amount of MTA, its pharmaceutically acceptable salts and/or prodrugs thereof.

The present invention further relates to the use of MTA, its pharmaceutically acceptable salts and/or prodrugs thereof as active ingredient in the manufacture of a medicament for the regeneration of nerve cells. In other words, the present invention also relates to MTA, its pharmaceutically acceptable salts and/or prodrugs thereof for use in the regeneration of nerve cells. Similarly, the present invention also relates to a method of regenerating nerve cells comprising administering to a subj ect in need thereof an effective amount of MTA or of one of its pharmaceutically acceptable salts and/or prodrugs thereof.

The present invention further relates to the use of MTA, its pharmaceutically acceptable salts and/or prodrugs thereof as active ingredient in the manufacture of a medicament for the prevention or treatment of a neurological or psychiatric disease. In other words, the present invention also relates to MTA, its pharmaceutically acceptable salts and/or prodrugs thereof for use in the prevention or treatment of a neurological or psychiatric disease. Similarly, the present invention also relates to a method of prevention or treatment of a neurological or psychiatric disease comprising administering to a subject in need thereof an effective amount of MTA, its pharmaceutically acceptable salts and/or prodrugs thereof.

### Description of the figures

Figure 1: MTA toxicity profile in mixed cultures of neurons and astrocytes from the cerebral cortex. MTA toxicity profile shows that the compound is innocuous at the concentrations assayed in mixed cultures of neurons and astrocytes from the cerebral cortex. (Left) Viability assays using calcein-AM. (Right) Lactate dehydrogenase (LDH) release assays. In both instances, MTA is not toxic to neuron-astrocyte co-cultures at the concentrations assayed.

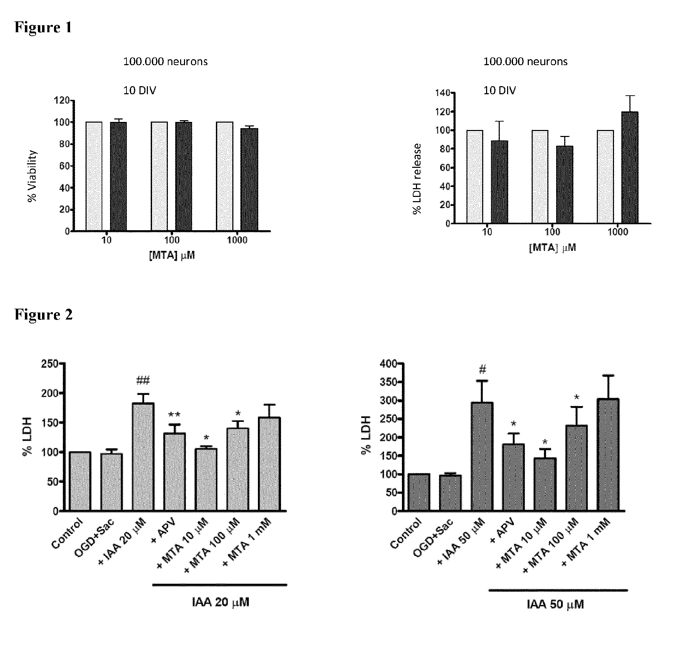

Figure 2: MTA protects from simulated ischemia in mixed cultures of neurons and astrocytes obtained from the cerebral cortex. Oxygen-glucose deprivation (OGD)-induced damage and protection by MTA in mixed cultures of neurons and astrocytes obtained from the cerebral cortex. Two ischemia conditions were assayed using iodoacetate (IAA) at 20 or 50 µM (left and right, respectively). In both conditions, MTA was highly protective. For comparison, (2R)-amino-5-phosphonovaleric acid (APV), an antagonist of the NMDA receptor, was also assayed. MTA at 10 µM was even more protective than APV (50 µM). * or # p< 0.05; ** or ## p<0.01.
Figure 3: Treatment with MTA of rats after focal ischemia. MTA does not protect from brain tissue damage after 90 min of occlusion of the middle cerebral artery (MCAO). Treatment with MTA started at 30 min of the onset of ischemia (i.p., twice daily). Rats were sacrificed at 3 days of reperfusion, cut in slices and stained with 2,3,5-triphenyl-2H-tetrazolium chloride (TTC) to view damaged region. Values represent average ± Standard error of the mean (SEM) of the damaged area.
Figure 4: Treatment with MTA of rats after global ischemia. MTA protects CA1 hippocampal neurons from cell death after 10 min global ischemia. Treatment with MTA started at 30 min post-ischemia (i.p., twice daily). Rats were sacrificed at 7 days of reperfusion, sectioned and stained with Fluoro-jade to view dying cells. Values represent average ± SEM of fluorescence in arbitrary units (AU), P<0.01.
Figure 5: MTA toxicity profile in oligodendrocytes and protection from excitotoxic insults. MTA toxicity profile in cultured oligodendrocytes derived from the optic nerve (A). (B) MTA protects oligodendrocytes from low intensity excitotoxic insults with AMPA (10 µM). However, it does not protect oligodendrocytes from death at high AMPA dosages (100 µM).
Figure 6: MTA protects from simulated ischemia in optic nerves. OGD-induced damage in isolated optic nerve was attenuated by the AMPA/kainate and NMDA receptor antagonists 6-cyano-7-nitro-quinoxaline-2,3-dione (CNQX) and APV, alone or together (top). In addition, MTA was also protective (bottom). *p< 0.05; **p<0.01.
Figure 7: Time-course of NMDA-mediated neurotoxicity in a pure neuronal culture. Data represent mean + s.e. of 12 experiments. ***p<0.01 as compared to vehicle.
Figure 8: Concentration-response curve of NMDA-mediated neurotoxicity in a pure neuronal culture. Data represent mean + s.e. of 12 experiments. ***p<0.01 as compared to vehicle.
Figure 9: Time-course of NMDA-mediated increase in caspase 3 activity in a pure neuronal culture. Data represent mean + s.e. of 12 experiments. **p<0.05 as compared to vehicle.
Figure 10: Lack of effect of MTA (100 to 500 µM) on NMDA-mediated toxicity in a pure neuronal culture. Data represent mean + s.e. of 12 experiments. MK (MK-801, 10 µM) ***p<0.001 as compared to NMDA.
Figure 11: Lack of effect of MTA (100 to 500 µM) on NMDA-mediated increase in caspase 3 activity in a pure neuronal culture. Data represent mean + s.e. of 12 experiments. MK (MK-801, 10 µM) ***p<0.001 as compared to vehicle and NMDA.
Figure 12: Effect of MTA (250 µM) on NMDA-mediated increase in caspase 3 activity in a mixed neuronal-glial culture. Data represent mean + s.e. of 12 experiments. MK (MK-801, 10 µM) ***p<0.001 as compared to vehicle and NMDA.
Figure 13.I: Neuroinflammation model in cerebellar organotypic cultures.
   A) Immunostaining for myelin basic protein (MBP) (green) and neurofilament subunit (NFL) (red) obtained by confocal microscopy. Control cultures show myelin sheaths (in green) often aligning with axons (panel a and d). Immunostaining after 15 µg/ml lipopolysaccharide (LPS) treatment shows only few myelinated neurofilaments (panel c and f), whereas organotypic cultures treated with 5 µg/ml of LPS shows more preserved myelination and less axonal swelling (panel b and e). In panels g-i is shown neurofilament-light staining. Cerebellar organotypic cultures treated with 15 µg/ml LPS shown axonal swelling (arrows in panel i) and axonal transecting (arrowheads in panel i) absent in control and in organotypic cultures treated with 5 µg/ml of LPS. Organotypic cultures stained after 24 hrs of treatment. Scale bar 50 µm (in panels a-c), 10 µm (in panels d-f) and 20 µm (in panels g-i). B) TNF-alpha concentration measured by ELISA. LPS treatments (5 µg/ml and 15 µg/ml) induce a release in the medium peaking at 3 hrs of TNF-alpha. C) Western blot analysis for CNPase, APP, iNOS and GAPDH. 15 µg/ml of LPS induce demyelination (first panel top) axonal damage (second panel top), and oxidative stress (iNOS in thirst panel top) at 24 hrs of treatment and GAPDH increase start up 1 hr until 96 hrs. 5 µg/ml induce less demyelination (first panel bottom) and slightly axonal damage at 12 hrs (second panel bottom). Oxidative stress (iNOS and DAPDH) shown the same increase (thirst panel bottom and fours panel bottom) that in organotypic culture treated with 15 µg/ml.

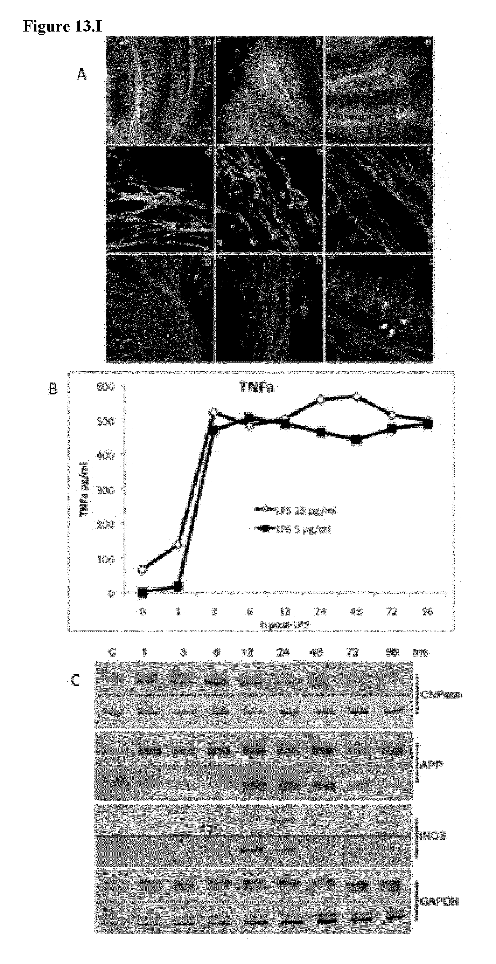

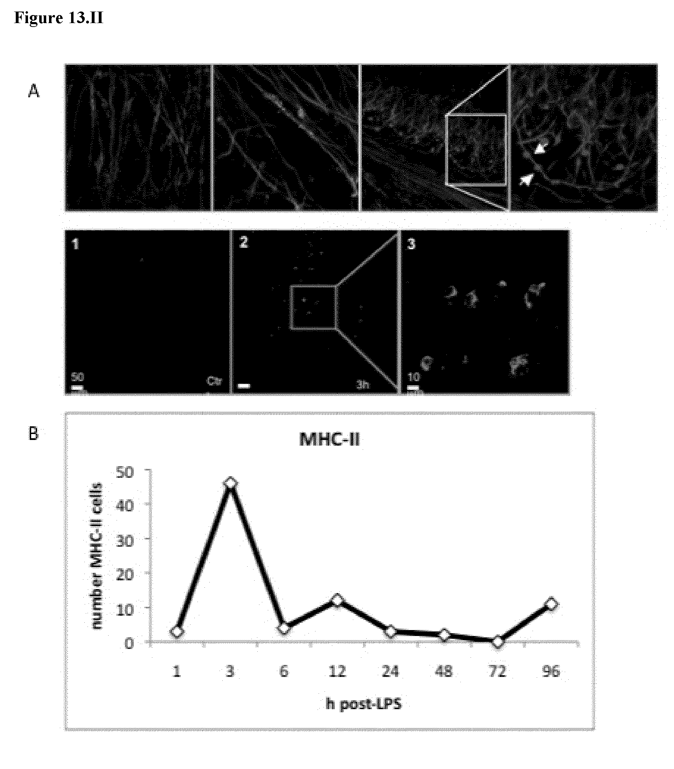

Figure 13.II: Activation of microglia in mouse cerebellar organotypic culture. A) Labeling for MHCII seen with confocal microscopy. On panel 2 a higher number of positive cells for MHCII in respect with the control not treated with LPS (panel 1) can be observed. On panel 3 an increase in panel 2 is observed. B) The graph shows how the treatment with LPS induces the activation of MHCII at the three hours after treatment.

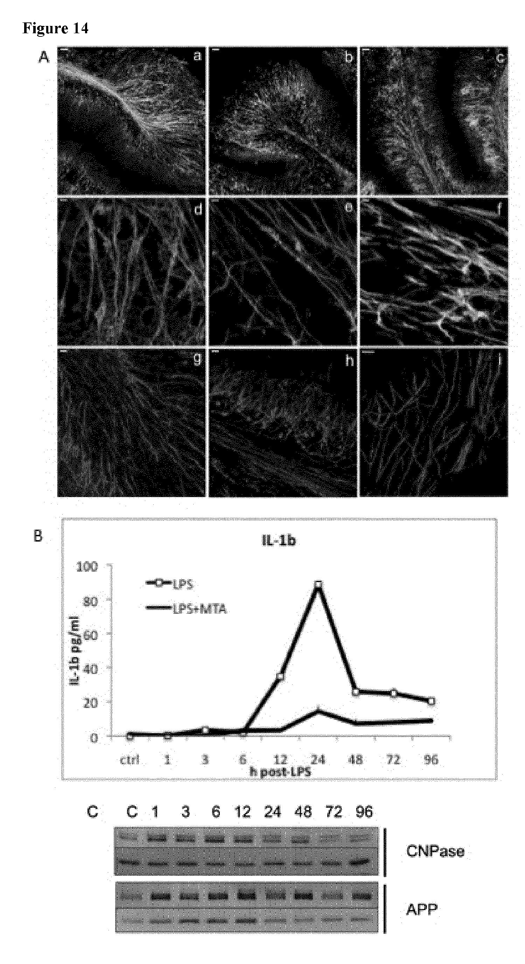

Figure 14: MTA as neuroprotector in a neuroinflammation model.
   A) Immunostaining for MBP (green) and NFL (red) obtained by confocal microscopy. Control cultures show myelin sheaths (in green) often aligning with axons (panel a and d). Immunostaining after 15 µg/ml LPS treatment shows only few myelinated neurofilaments (panel b and e), whereas organotypic cultures treated with 192 µM of MTA shows myelin sheath often aligning with axon (panel c and f). In panels g-i is shown neurofilament-light staining. Cerebellar organotypic cultures treated with 15 µg/ml LPS shown axonal swelling (arrow in panel h) and axonal transecting (arrowhead in panle h) absent in control and in organotypic cultures treated with MTA (panels g and i). Organotypic cultures stained after 24 hrs of treatment. Scale bar 50 µm (in panels a-c), 5 µm (in panels d-f) and 20 µm (in panels g-i). B) IL1-beta concentration measured by ELISA. LPS treatments induce a release in the medium peaking at 3 hrs. MTA treatment induce minor quantity II,1-beta release at 3 hrs. C) Western blot analysis for CNPase and APP. In neuroinflammation model, 15 µg/ml of LPS induce demyelination (first panel top) and axonal damage (second panel top) at 24 hrs of treatment absents in organotypic cultures treated with MTA (first and second panel bottom).

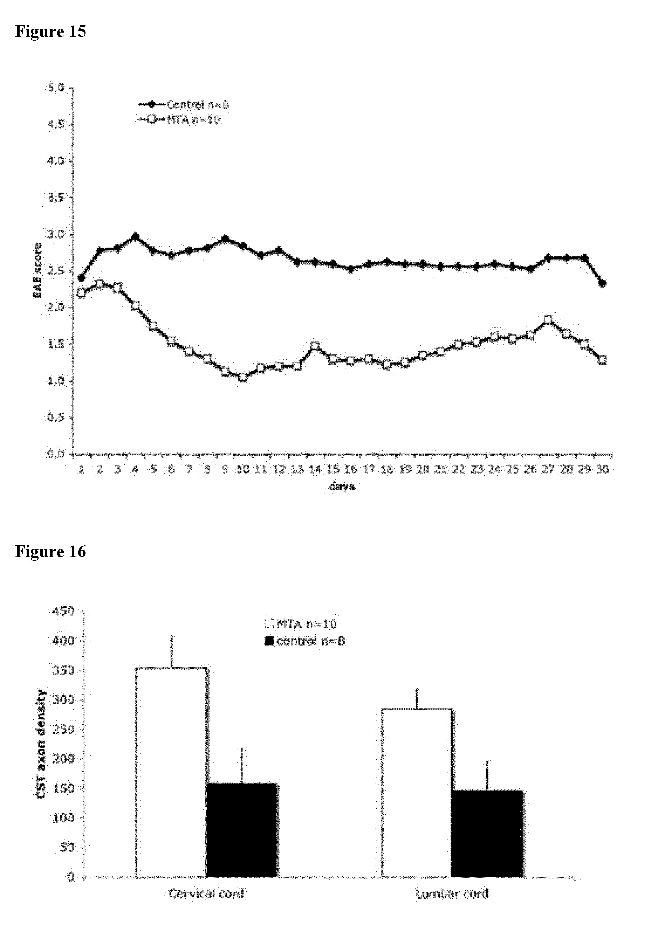

Figure 15: Clinical score of C57B6 mice suffering chronic EAE and treated with MTA after disease onset. Day 1: first day with an EAE score of 2 or higher; Placebo (black squares), MTA 96 µMol/Kg (open squares).
Figure 16: Axonal density quantification (as described in methods) in the placebo (black) or MTA (white) treated animals in the two locations in the spinal cord (cervical and lumbar spinal cord).

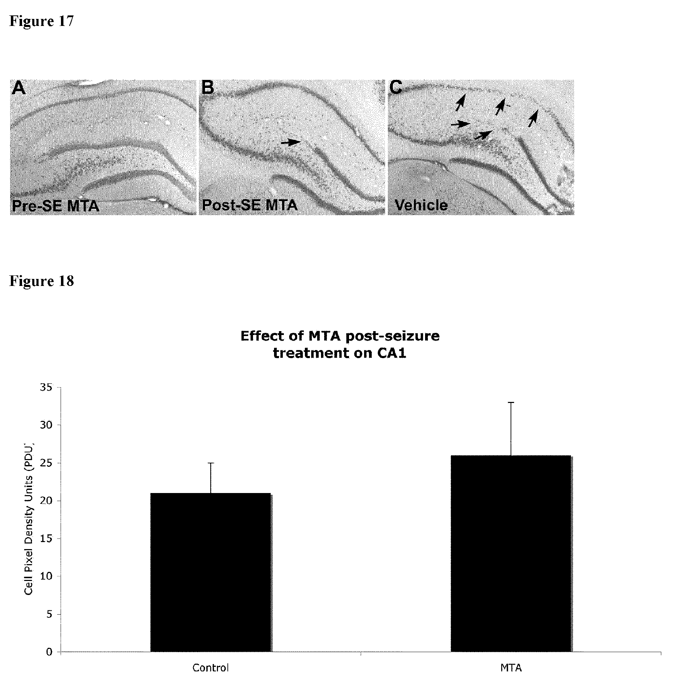

Figure 17. Representative examples of hippocampal NeuN immunoreactivity at 30 days after pilocarpine-induced status epilepticus (SE) in adult rats treated with the pre-SE MTA (A), post-SE MTA (B) or vehicle (C) regimens. A, The pre-SE MTA subject shows good preservation of neurons in the dentate gyrus and pyramidal cell layer, while the rat in the post-SE MTA group shows only mild cell loss in the dentate gyrus (arrow in B). The control shows severe cell loss in both the dentate gyrus and area CA1 pyramidal cell layer (C).
Figure 18. Effect of post-SE MTA on rat hippocampal CA1 region. A 90-minute SE episode was induced in adult rats with pilocarpine treatment and terminated with diazepam. Immediately following termination of SE, MTA treatment was initiated daily for 3 days. The rats were sacrificed 28 days after the end of MTA treatment. Control rats received Tris-buffer in place of MTA. Means ± SEMs are shown. A non-significant trend toward increased cell numbers in the MTA group was found (*p*=0.057).

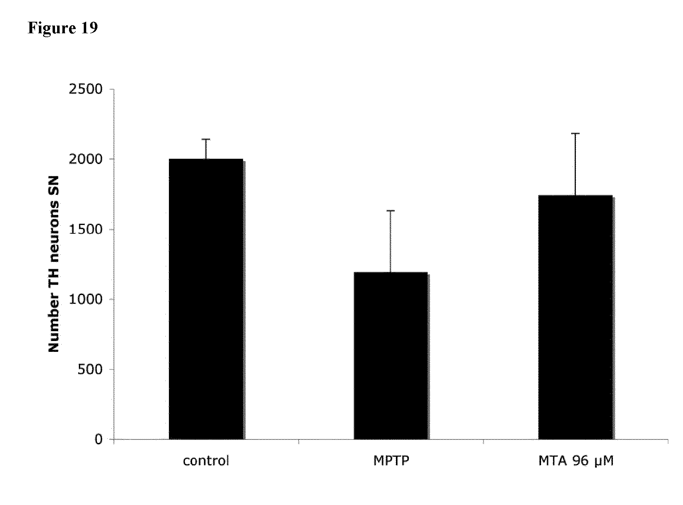

Figure 19. Effect of MTA on the survival of dopaminergic neurons (TH cells) in the animal model of Parkinson disease, the C57B6 mice treated with MPTP. Control animals have a normal density of TH cells in their substantia nigra. Animals treated with MPTP have a decrease in the number of TH neurons by 40% compared to controls. By contrast, animals treated with MTA (96 µM) after being exposed to MPTP have a non-significant reduction in the number of TH neurons compared to controls (10%) and significantly higher number of TH neurons that MPTP animals.

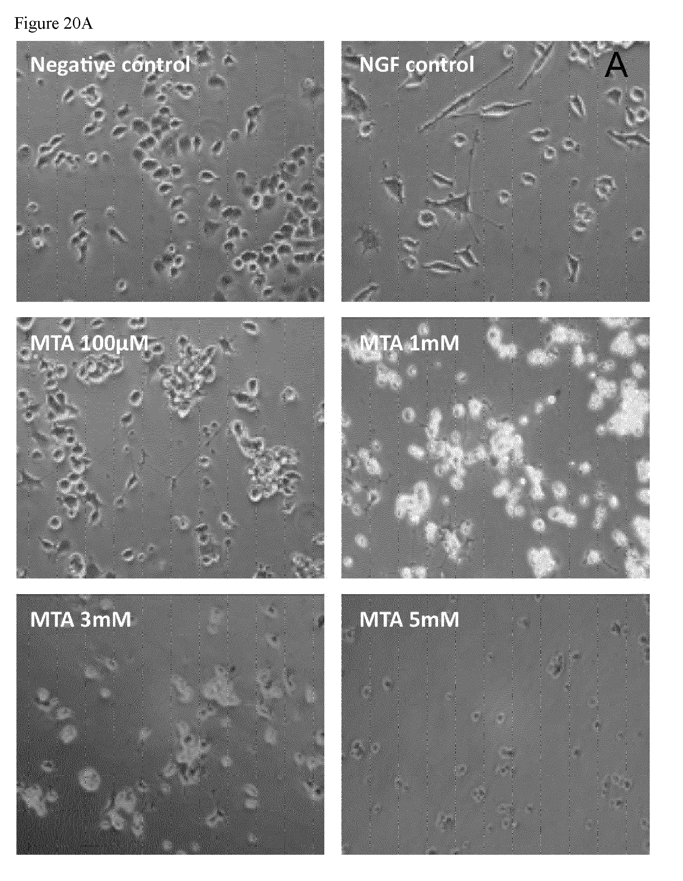

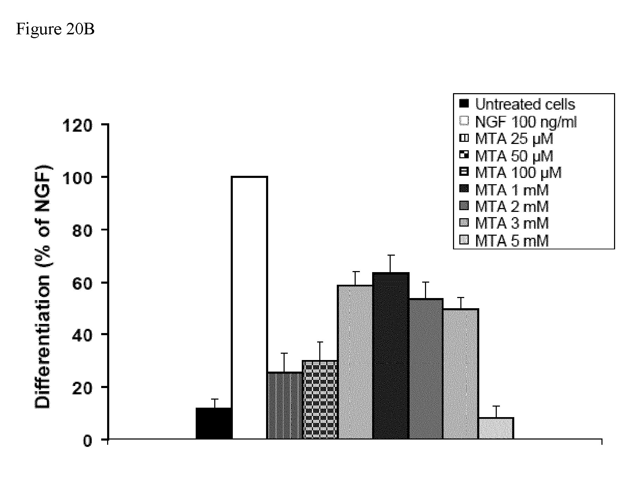

Figure 20. Effect of MTA on neuronal differentiation. A) PC12 cells treated for four days with NGF (100 ng/ml) or MTA at different concentrations. Differences in neurite development were observed with the different treatments. B) Quantification of neurites in PC12 cells treated with NGF (100 ng/ml) or different MTA concentrations for four days. The results are expressed as a percentage of differentiation with respect to control by NGF.

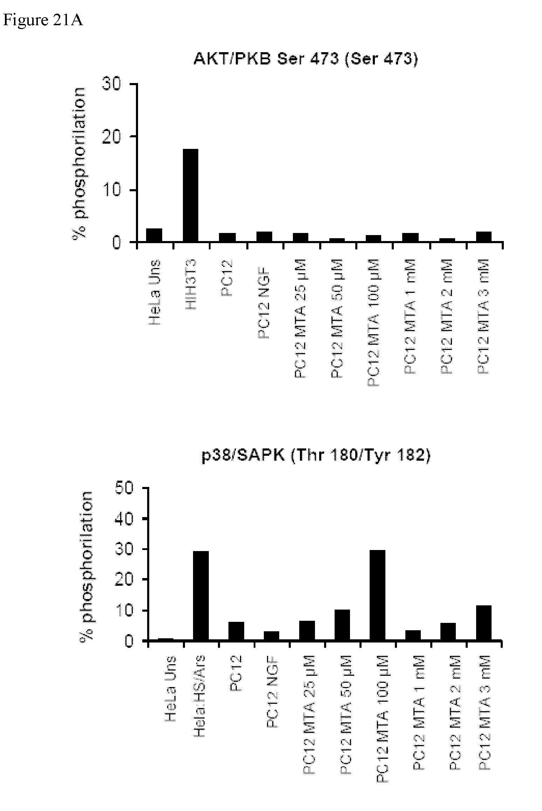

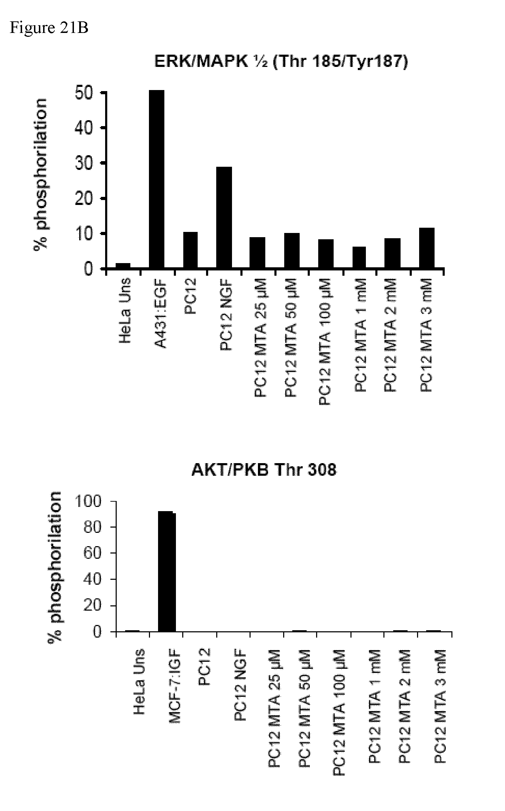

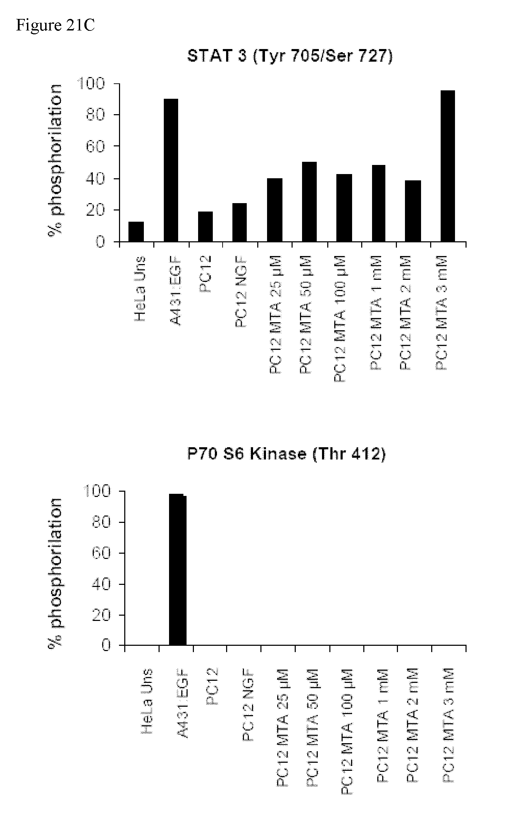

Figure 21. Quantification of the percentage of phosphorylation of different intracellular proteins in PC12 cells treated with NGF (100 ng/ml) or MTA at different concentrations for four days using Luminex technology.

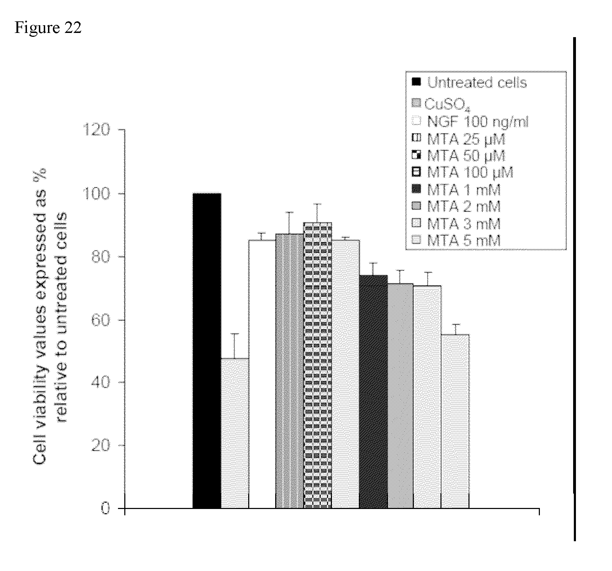

Figure 22. Percentage of cell viability (MTT assay) in RN22 cells exposed to stress with sulphate copper (150 µM) and treated with NGF (100 ng/ml) or different MTA concentrations for twenty-four hours.

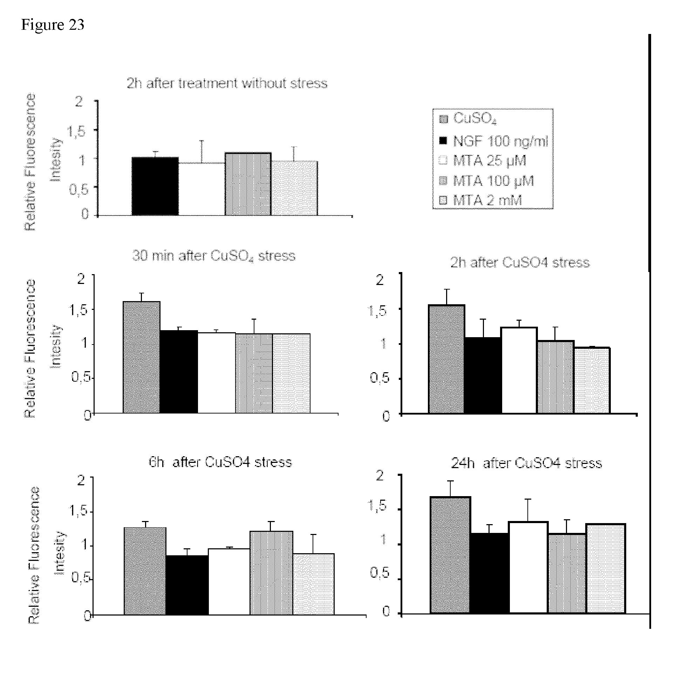

Figure 23. Determination of ROS levels in RN22 cells exposed to stress with sulphate copper (150 µM) and treated with NGF (100 ng/ml) or different MTA concentrations for different time periods.

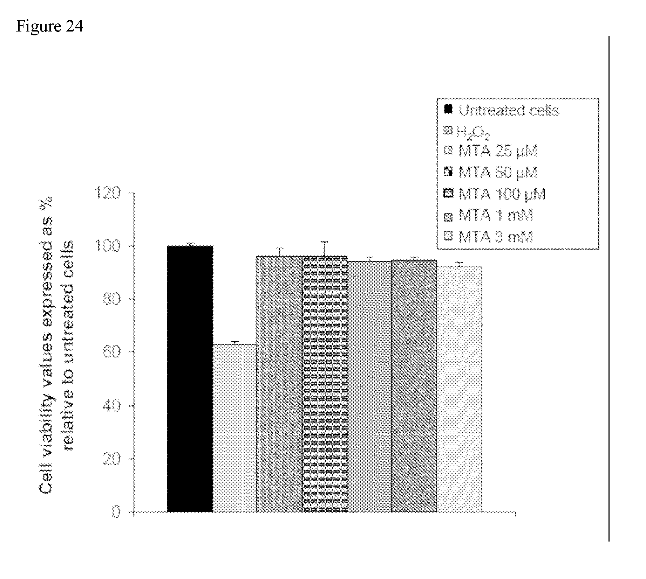

Figure 24. Percentage of cell viability (MTT assay) in RN22 cells exposed to stress with hydrogen peroxide (100 µM) and treated with different MTA concentrations for twenty-four hours.

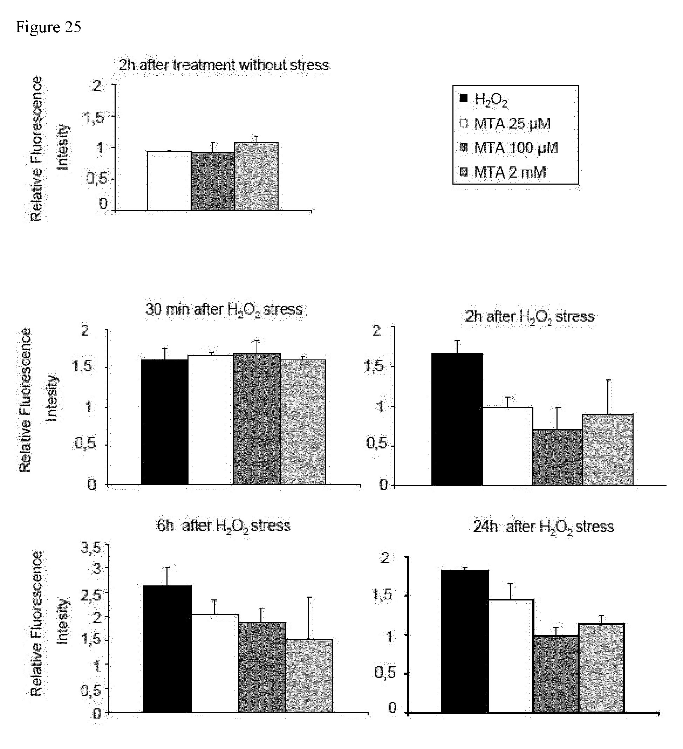

Figure 25. Determination of ROS levels in RN22 cells exposed to stress with hydrogen peroxide (100 µM) and treated with different MTA concentrations for different periods of time.

### Disclosure of the invention

The present invention relates to the use of MTA, its pharmaceutically acceptable salts and/or prodrugs thereof as active ingredient in the manufacture of a medicament for the prevention or treatment of nerve cell death or damage. In other words, the present invention relates to MTA, its pharmaceutically acceptable salts and/or prodrugs thereof for use in the prevention or treatment of nerve cell death or damage. Similarly, the present invention relates to a method of neuroprotection comprising administering to a subj ect in need thereof an effective amount of MTA, its pharmaceutically acceptable salts and/or prodrugs thereof.

The present invention also relates to the use of MTA, its pharmaceutically acceptable salts and/or prodrugs thereof as active ingredient in the manufacture of a neuroprotective medicament. In other words, the present invention also relates to MTA, its pharmaceutically acceptable salts and/or prodrugs thereof for use as a neuroprotective drug. Similarly, the present invention also relates to a method of prevention or treatment of nerve cell death or damage comprising administering to a subject in need thereof an effective amount of MTA , its pharmaceutically acceptable salts and/or prodrugs thereof.

MTA, also referred herein as 5'-methylthioadenosine, is a commercial product that can be provided by for example the company Sigma. Alternatively, this compound can be obtained by known methods to the skilled in the art, for example, from S-adenosyl-methionine (SAM) according to the procedure described by Schlenk F. et al., Arch. Biochem. Biophys., 1964, 106:95-100. The CAS registry number of MTA is 2457-80-9, and its structural formula is:

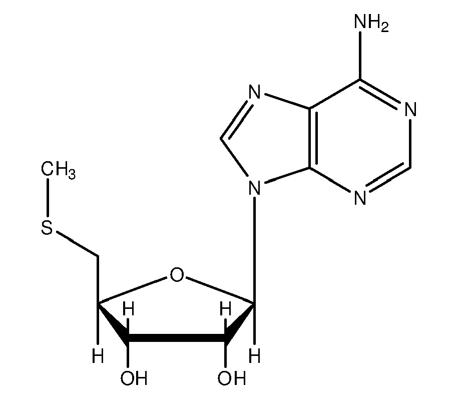

The term "prodrug", as used herein, includes any compound derived from MTA, for example, the ester, amide, phosphate, etc., which, upon being administered to an individual, is capable of providing MTA or the pharmaceutically acceptable salt thereof, directly or indirectly, to said individual. Preferably, said derivative is a compound that increases the bioavailability of MTA when administered to an individual or that promotes the release of MTA in a biological compartment. The nature of said derivative is not critical, provided that it may be administered to an individual and that it provides MTA in an individual's biological compartment. The preparation of said prodrug may be performed by conventional methods known by those skilled in the art. Prodrugs of MTA may for instance be conveniently prepared by attaching a promoiety to any one or both of the hydroxyl groups of the ribose ring. An example of an MTA prodrug is 2'-[(2Z)-3-(4-hydroxyphenyl)-2-methoxy-2-propenoate]-3'-[(2E)-3-(1H-imidazol-4-yl)-2-propenoate]-5'-S-methyl-5'-thio-adenosine (Journal of Medicinal Chemistry, 47(9):2243-2255, 2004). Another example of a prodrug or precursor of MTA is S-adenosylmethionine (SAM).

The term "pharmaceutically acceptable" means that a compound or combination of compounds is sufficiently compatible with the other ingredients of a formulation, and not deleterious to the patient up to those levels acceptable by the industry standards.

For therapeutic use, salts of 5'-methylthioadenosine are those wherein the counter-ion is pharmaceutically acceptable.

The term salt as mentioned herein is meant to comprise any stable salts, which the MTA is able to form. Preferred are the pharmaceutically acceptable salts. Salts that are not pharmaceutically acceptable are also embraced in the scope of the present invention, since they refer to intermediates that may be useful in the preparation of compounds with pharmacological activity.

The salts can conveniently be obtained by treating the base form of MTA with such appropriate acids as inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids.

The pharmaceutically acceptable salts can be obtained by treating the base form of MTA with such appropriate pharmaceutically acceptable acids like inorganic acids, for example, hydrohalic acids, including hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propane-tricarboxylic, methanesulfonic, ethanesulfonic, benzenesulfonic, 4-methylbenzene-sulfonic, cyclohexanesulfamic, 2-hydroxybenzoic, 4-amino-2-hydroxybenzoic and the like acids.

Conversely the salt form can be converted by treatment with alkali into the free base form.

The term "preventing" refers to keep from happening, existing, or alternatively delaying the onset or recurrence of a disease, disorder, or condition to which such term applies, or of one or more symptoms associated with a disease, disorder, or condition. The term "prevention" refers to the act of preventing, as "preventing" is defined immediately above.

The term "treating", as used herein, refers to reversing, alleviating, or inhibiting the progress of the disorder or condition to which such term applies, or one or more symptoms of such disorders or condition. The term "treatment" refers to the act of treating, as "treating" is defined immediately above.

The term "subj ect" means animals, in particular mammals such as dogs, cats, cows, horses, sheep, geese, and humans. Particularly preferred subjects are mammals, including humans of both sexes.

An "effective amount" of MTA and pharmaceutically acceptable salts thereof, may be in the range from 0.01 mg to 50 g per day, from 0.02 mg to 40 g per day, from 0.05 mg to 30 g per day, from 0.1 mg to 20 g per day, from 0.2 mg to 10 g per day, from 0.5 mg to 5 g per day, from 1 mg to 3 g per day, from 2 mg to 2 g per day, from 5 mg to 1,5 g per day, from 10 mg to 1 g per day, from 10 mg to 500 mg per day.

Nerve cells include those cells from any region of the brain, spinal cord, optic nerve, retina, and peripheral ganglia. Neurons include those in embryonic, fetal, or adult neural tissue, including tissue from the hippocampus, cerebellum, spinal cord, cortex (e.g., motor or somatosensory cortex), striatum, basal forebrain (cholinergic neurons), ventral mesencephalon (cells of the substantia nigra), and the locus ceruleus (neuroadrenaline cells of the central nervous system).

In one embodiment of the present invention, MTA, its pharmaceutically acceptable salts and/or prodrugs thereof may be used for the prevention or treatment of one or more, preferably two or more, pathological or harmful conditions related to nerve cell death or damage selected from, but not being limited to, chemical substances such as oxidative stress conditions, toxic substances, infectious organisms, radiation, traumatic injury, hypoxia, ischemia, abnormal misfolded proteins, excitotoxins, free radicals, endoplasmic reticulum stressors, mitochondrial stressors including but not limited to inhibitors of the electron transport chain, Golgi apparatus antagonists, axonal damage or loss, demyelination, inflammation, pathological neuronal burst (seizures). Also preferably, the uses and methods of the present invention are directed to preventing or treating nerve cell death or damage, regardless of cause.

The terms "neuroprotection", "neuroprotective", or "neuroprotective effect" refer to the ability to prevent or reduce death or damage to nerve cells, including neurons and glia, or rescuing, resuscitating or reviving nerve cells, e.g., following in pathological or harmful conditions to the brain, central nervous system or peripheral nervous system. Thus, this neuroprotective effect comprises the conferred ability of neuronal cells to maintain or recover their neuronal functions. It stabilizes the cell membrane of a neuronal cell or helps in the normalization of neuronal cell functions. It prevents the loss of viability or functions of neuronal cells. It comprises the inhibition of progressive deterioration of neurons that leads to cell death. It refers too to any detectable protection of neurons from stress. Neuroprotection includes the regeneration of nerve cells, i.e. the re-growth of a population of nerve cells after disease or trauma.

Currently the majority of the neurological and psychiatric diseases lack specific treatments aimed to stop or ameliorate the course of the disease, which are called "disease modifying drugs". These disease modifying drugs contrast with the symptomatic therapy that is commonly used for such diseases but does not change the course of the disease. A neuroprotective drug is a Disease Modifying Drug (DMD) for the treatment of brain diseases.

As such, in one embodiment, the present invention relates to the use of MTA, its pharmaceutically acceptable salts and/or prodrugs thereof as active ingredient in the manufacture of a medicament for the regeneration of nerve cells. In other words, the present invention relates to MTA, its pharmaceutically acceptable salts and/or prodrugs thereof for use for the regeneration of nerve cells. Similarly, the present invention relates to a method of regenerating nerve cells comprising administering to a subject in need thereof an effective amount of MTA, its pharmaceutically acceptable salts and/or prodrugs thereof.

Neuroprotection may be determined directly by, for example, measuring the delay or prevention of neuronal death, such as, for example, by a reduction in the number of apoptotic neurons in cerebrocortical cultures following a stress. Neuroprotection may also be determined directly by, for example, measuring the severity or extent of damage to, or functional loss by, a tissue or organ of the nervous system following such a stress, such as, for example, by measuring a decrease in the size of brain infarcts after occlusion of the middle cerebral artery (MCAO) or reperfusion injury. Also, neuroprotection can be identified by magnetic resonance imaging (measuring brain volume, tractography, levels of N-acetyl-asparte by spectroscopy) or by retinal imaging with optic coherent imaging (retinal nerve fiber layer thining) or retinal spectroscopy (levels of cytochrome c, oxyhemoglobin, lactate, glutamate, iNOS). Alternatively, neuroprotection may be determined indirectly by detecting the activation of one or more biological mechanisms for protecting neurons, including, but not limited to, detecting activation of the Keapl/Nrf2 pathway or induction of one or more phase 2 enzymes, including but not limited to hemeoxygenase-1 (HO-1). Methods of detecting and measuring neuronal protection are provided in the Examples below, and other such methods are known in the art.

The various uses and methods employing MTA, its pharmaceutically acceptable salts and/or prodrugs thereof in the present invention comprise acute administration, i.e. occurring within several minutes to about several hours from injury, or chronic administration, suitable for chronic neurological or psychiatric diseases.

In one embodiment of the present invention, in the various uses and methods of neuroprotection or of prevention or treatment of nerve cell death or damage, or regeneration of nerve cells, MTA, its pharmaceutically acceptable salts and/or prodrugs thereof is administered to a subject with a neurological or psychiatric disease.

Neurological diseases are those disorders of the central and peripheral nervous system, including disorders of the brain, spinal cord, cranial nerves, peripheral nerves, nerve roots, autonomic nervous system, neuromuscular junction, and muscle.

Diseases of the central and peripheral nervous system include, without being limited to, as knowledge in clinical manifestations advances, Absence of the Septum Pellucidum, Acid Lipase Disease, Acid Maltase Deficiency, Acquired Epileptiform Aphasia, Acute Disseminated Encephalomyelitis, Adie's Pupil, Adie's Syndrome, Adrenoleukodystrophy, Agenesis of the Corpus Callosum, Agnosia, Aicardi Syndrome, Aicardi-Goutieres Syndrome Disorder, AIDS - Neurological Complications, Alexander Disease, Alpers' Disease, Alternating Hemiplegia, Alzheimer's Disease, Amyotrophic Lateral Sclerosis, Anencephaly, Aneurysm, Angelman Syndrome, Angiomatosis, Anoxia, Antiphospholipid Syndrome, Aphasia, Apraxia, Arachnoid Cysts, Arachnoiditis, Arnold-Chiari Malformation, Arteriovenous Malformation, Asperger Syndrome, Ataxia, Ataxia Telangiectasia, Ataxias and Cerebellar or Spinocerebellar Degeneration, Atrial Fibrillation and Stroke, Attention Deficit-Hyperactivity Disorder (ADHD), Autism, Autonomic Dysfunction, Back Pain, Barth Syndrome, Batten Disease, Becker's Myotonia, Behcet's Disease, Bell's Palsy, Benign Essential Blepharospasm, Benign Focal Amyotrophy, Benign Intracranial Hypertension, Bernhardt-Roth Syndrome, Binswanger's Disease, Blepharospasm, Bloch-Sulzberger Syndrome, Brachial Plexus Birth Injuries, Brachial Plexus Injuries, Bradbury-Eggleston Syndrome, Brain and Spinal Tumors, Brain Aneurysm, Brain infarction, Brain ischemia, Brain Injury, Brown-Sequard Syndrome, Bulbospinal Muscular Atrophy, CADASI, Canavan Disease, Carpal Tunnel Syndrome, Causalgia, Cavernomas, Cavernous Angioma, Cavernous Malformation, Central Cervical Cord Syndrome, Central Cord Syndrome, Central Pain Syndrome, Central Pontine Myelinolysis, Cephalic Disorders, Ceramidase Deficiency, Cerebellar Degeneration, Cerebellar Hypoplasia, Cerebral Aneurysm, Cerebral Arteriosclerosis, Cerebral Atrophy, Cerebral Beriberi, Cerebral Cavernous Malformation, Cerebral Gigantism, Cerebral Hypoxia, Cerebral Palsy, Cerebro-Oculo-Facio-Skeletal Syndrome, Charcot-Marie-Tooth Disease, Chiari Malformation, Cholesterol Ester Storage Disease, Chorea, Choreoacanthocytosis, Chronic Inflammatory Demyelinating Polyneuropathy (CIDP), Chronic Orthostatic Intolerance, Chronic Pain, Cockayne Syndrome Type II, Coffin Lowry Syndrome, COFS, Colpocephaly, Coma, Complex Regional Pain Syndrome, Congenital Facial Diplegia, Congenital Myasthenia, Congenital Myopathy, Congenital Vascular Cavernous Malformations, Corticobasal Degeneration, Cranial Arteritis, Craniosynostosis, Creutzfeldt-Jakob Disease, Cumulative Trauma Disorders, Cushing's Syndrome, Cytomegalic Inclusion Body Disease, Cytomegalovirus Infection, Dancing Eyes-Dancing Feet Syndrome, Dandy-Walker Syndrome, Dawson Disease, De Morsier's Syndrome, Deep Brain Stimulation for Parkinson's Disease, Dejerine-Klumpke Palsy, Dementia, Dementia - Multi-Infarct, Dementia - Semantic, Dementia - Subcortical, Dementia With Lewy Bodies, Dentate Cerebellar Ataxia, Dentatorubral Atrophy, Dermatomyositis, Developmental Dyspraxia, Devic's Syndrome, Diabetic Neuropathy, Diffuse Sclerosis, Dravet Syndrome, Dysautonomia, Dysgraphia, Dyslexia, Dysphagia, Dyspraxia, Dyssynergia Cerebellaris Myoclonica, Dyssynergia Cerebellaris Progressiva, Dystonias, , Early Infantile Epileptic Encephalopathy, Empty Sella Syndrome, Encephalitis, Encephalitis Lethargica, Encephaloceles, Encephalopathy, Encephalopathy, familial infantile, with intracranial calcification and chronic cerebrospinal fluid lymphocytosis; Cree encephalitis; Pseudo-Torch syndrome; Pseudotoxoplasmosis syndrome, Encephalotrigeminal Angiomatosis, Epilepsy, Epileptic Hemiplegia, Erb-Duchenne and Dejerine-Klumpke Palsies, Erb's Palsy, Essential Tremor, Extrapontine Myelinolysis, Fabry Disease, Fahr's Syndrome, Fainting, Familial Dysautonomia, Familial Hemangioma, Familial Idiopathic Basal Ganglia Calcification, Familial Periodic Paralyses, Familial Spastic Paralysis, Farber's Disease, Febrile Seizures, Fibromuscular Dysplasia, Fisher Syndrome, Floppy Infant Syndrome, Foot Drop, Friedreich's Ataxia, Frontotemporal Dementia, Gangliosidoses, Gaucher's Disease, Gerstmann's Syndrome, Gerstmann-Straussler-Scheinker Disease, Giant Axonal Neuropathy, Giant Cell Arteritis, Giant Cell Inclusion Disease, Globoid Cell Leukodystrophy, Glossopharyngeal Neuralgia, Glycogen Storage Disease, Guillain-Barré Syndrome, Hallervorden-Spatz Disease, Head Injury, Headache, Hemicrania Continua, Hemifacial Spasm, Hemiplegia Alterans, Hereditary Neuropathies, Hereditary Spastic Paraplegia, Heredopathia Atactica Polyneuritiformis, Herpes Zoster, Herpes Zoster Oticus, Hirayama Syndrome, Holmes-Adie syndrome , Holoprosencephaly, HTLV-1 Associated Myelopathy, Hughes Syndrome, Huntington's Disease, Hydranencephaly, Hydrocephalus, Hydrocephalus - Normal Pressure, Hydromyelia, Hypercortisolism, Hypersomnia, Hypertonia, Hypotonia, Hypoxia, Immune-Mediated Encephalomyelitis, Inclusion Body Myositis, Incontinentia Pigmenti, Infantile Hypotonia, Infantile Neuroaxonal Dystrophy , Infantile Phytanic Acid Storage Disease, Infantile Refsum Disease, Infantile Spasms, Inflammatory Myopathies, Iniencephaly, Intestinal Lipodystrophy, Intracranial Cysts, Intracranial Hypertension, Isaac's Syndrome, Joubert Syndrome, Kearns-Sayre Syndrome, Kennedy's Disease, Kinsbourne syndrome, Kleine-Levin Syndrome, Klippel-Feil Syndrome, Klippel-Trenaunay Syndrome (KTS), Kluver-Bucy Syndrome, Korsakoffs Amnesic Syndrome, Krabbe Disease, Kugelberg-Welander Disease, Kuru, Lambert-Eaton Myasthenic Syndrome, Landau-Kleffner Syndrome, Lateral Femoral Cutaneous Nerve Entrapment, Lateral Medullary Syndrome, Learning Disabilities, Leigh's Disease, Lennox-Gastaut Syndrome, Lesch-Nyhan Syndrome, Leukodystrophy, Levine-Critchley Syndrome, Lewy Body Dementia, Lipid Storage Diseases, Lipoid Proteinosis, Lissencephaly, Locked-In Syndrome, Lou Gehrig's Disease, Lupus - Neurological Sequelae, Lyme Disease - Neurological Complications, Machado-Joseph Disease, Macrencephaly, Megalencephaly, Melkersson-Rosenthal Syndrome, Meningitis, Meningitis and Encephalitis, Menkes Disease, Meralgia Paresthetica, Metachromatic Leukodystrophy, Microcephaly, Migraine, Miller Fisher Syndrome, Mild Cognitive Impairment, Mini-Strokes, Mitochondrial Myopathies, Moebius Syndrome, Monomelic Amyotrophy, Motor Neuron Diseases, Moyamoya Disease, Mucolipidoses, Mucopolysaccharidoses, Multifocal Motor Neuropathy, Multi-Infarct Dementia, Multiple Sclerosis, Multiple System Atrophy, Multiple System Atrophy with Orthostatic Hypotension, Muscular Dystrophy, Myasthenia - Congenital, Myasthenia Gravis, Myelinoclastic Diffuse Sclerosis, Myoclonic Encephalopathy of Infants, Myoclonus, Myopathy, Myopathy - Congenital, Myopathy - Thyrotoxic, Myotonia, Myotonia Congenita, Narcolepsy, Neuroacanthocytosis, Neurodegeneration with Brain Iron Accumulation, Neurofibromatosis, Neuroleptic Malignant Syndrome, Neurological Complications of AIDS, Neurological Complications Of Lyme Disease, Neurological Consequences of Cytomegalovirus Infection, Neurological Manifestations of Pompe Disease, Neurological Sequelae Of Lupus, Neuromyelitis Optica , Neuromyotonia, Neuronal Ceroid Lipofuscinosis, Neuronal Migration Disorders, Neuropathy - Hereditary, Neurosarcoidosis, Neurotoxicity, Nevus Cavernosus, Niemann-Pick Disease, Normal Pressure Hydrocephalus, Occipital Neuralgia, Ohtahara Syndrome, Olivopontocerebellar Atrophy, Opsoclonus Myoclonus, Orthostatic Hypotension, O'Sullivan-McLeod Syndrome, Overuse Syndrome, Pain - Chronic, Pantothenate Kinase-Associated Neurodegeneration, Paraneoplastic Syndromes, Paresthesia, Parkinson's Disease, Paroxysmal Choreoathetosis, Paroxysmal Hemicrania, Parry-Romberg, Pelizaeus-Merzbacher Disease, Pena Shokeir II Syndrome, Perineural Cysts, Periodic Paralyses, Peripheral Neuropathy, Periventricular Leukomalacia, Persistent Vegetative State, Pervasive Developmental Disorders, Phytanic Acid Storage Disease, Pick's Disease, Pinched Nerve, Piriformis Syndrome, Pituitary Tumors, Polymyositis, Pompe Disease, Porencephaly, Postherpetic Neuralgia, Postinfectious Encephalomyelitis, Post-Polio Syndrome, Postural Hypotension, Postural Orthostatic Tachycardia Syndrome, Postural Tachycardia Syndrome, Primary Dentatum Atrophy, Primary Lateral Sclerosis, Primary Progressive Aphasia, Prion Diseases, Progressive Hemifacial Atrophy, Progressive Locomotor Ataxia, Progressive Multifocal Leukoencephalopathy, Progressive Sclerosing Poliodystrophy, Progressive Supranuclear Palsy, Prosopagnosia, Pseudotumor Cerebri, Ramsay Hunt Syndrome I (formerly known as), Ramsay Hunt Syndrome II (formerly known as), Rasmussen's Encephalitis, Reflex Sympathetic Dystrophy Syndrome, Refsum Disease, Refsum Disease - Infantile, Repetitive Motion Disorders, Repetitive Stress Injuries, Restless Legs Syndrome, Retrovirus-Associated Myelopathy, Rett Syndrome, Reye's Syndrome, Rheumatic Encephalitis, Riley-Day Syndrome, Sacral Nerve Root Cysts, Saint Vitus Dance, Salivary Gland Disease, Sandhoff Disease, Schilder's Disease, Schizencephaly, Seitelberger Disease, Seizure Disorder, Semantic Dementia, Septo-Optic Dysplasia, Severe Myoclonic Epilepsy of Infancy (SMEI), Shaken Baby Syndrome, Shingles, Shy-Drager Syndrome, Sjögren's Syndrome, Sleep Apnea, Sleeping Sickness, Sotos Syndrome, Spasticity, Spina Bifida, Spinal Cord Infarction, Spinal Cord Injury, Spinal Cord Tumors, Spinal Muscular Atrophy, Spinocerebellar Atrophy, Spinocerebellar Degeneration, Steele-Richardson-Olszewski Syndrome, Stiff-Person Syndrome, Striatonigral Degeneration, Stroke, Sturge-Weber Syndrome, Subacute Sclerosing Panencephalitis, Subcortical Arteriosclerotic Encephalopathy, SUNCT Headache, Swallowing Disorders, Sydenham Chorea, Syncope, Syphilitic Spinal Sclerosis, Syringohydromyelia, Syringomyelia, Systemic Lupus Erythematosus, Tabes Dorsalis, Tardive Dyskinesia, Tarlov Cysts, Tay-Sachs Disease, Temporal Arteritis, Tethered Spinal Cord Syndrome, Thomsen's Myotonia, Thoracic Outlet Syndrome, Thyrotoxic Myopathy, Tic Douloureux, Todd's Paralysis, Tourette Syndrome, Transient Ischemic Attack, Transmissible Spongiform Encephalopathies, Transverse Myelitis, Traumatic Brain Injury, Tremor, Trigeminal Neuralgia, Tropical Spastic Paraparesis, Troyer Syndrome, Tuberous Sclerosis, Vascular Erectile Tumor, Vasculitis Syndromes of the Central and Peripheral Nervous Systems , Von Economo's Disease, Von Hippel-Lindau Disease (VHL), Von Recklinghausen's Disease, Wallenberg's Syndrome, Werdnig-Hoffman Disease, Wernicke-Korsakoff Syndrome, West Syndrome, Whiplash, Whipple's Disease, Williams Syndrome, Wilson's Disease, Wolman's Disease, X-Linked Spinal and Bulbar Muscular Atrophy, Zellweger Syndrome, optic neuritis, Chronic fatigue syndrome, fibromialgia, psychiatric diseases such as mood disorders, major depression, bipolar syndrome, psycosis, eschizophrenia, obsessive-compulsive-syndrome, etc., Toxic or drug abuse diseases such as alcoholism and drug abuse, Encephalopathy like hepatic encephalopathy.

Psychiatric disorders include those listed by the Diagnostic and Statistical Manual of Mental Disorders, Fourth Edition (DSM-IV) published by the American Psychiatric Association, and covers all mental health disorders for both children and adults. In particular, psychiatric disorders include a disorder selected from Acute Stress Disorder; Adjustment Disorder Unspecified; Adjustment Disorder with Anxiety; Adjustment Disorder with Depressed Mood; Adjustment Disorder with Disturbance of Conduct; Adjustment Disorder with Mixed Anxiety and Depressed Mood; Adjustment Disorder with Mixed Disturbance of Emotions and Conduct; Agoraphobia without History of Panic Disorder; Anorexia Nervosa; Antisocial Personality Disorder; Anxiety Disorder Due to Medical Condition; Anxiety Disorder, NOS; Avoidant Personality Disorder; Bipolar Disorder NOS; Bipolar I Disorder, Most Recent Episode Depressed, In Full Remission; Bipolar I Disorder, Most Recent Episode Depressed, In Partial Remission; Bipolar I Disorder, Most Recent Episode Depressed, Mild; Bipolar I Disorder, Most Recent Episode Depressed, Moderate; Bipolar I Disorder, Most Recent Episode Depressed, Severe With Psychotic Features; Bipolar I Disorder, Most Recent Episode Depressed, Severe Without Psychotic Features; Bipolar I Disorder, Most Recent Episode Depressed, Unspecified; Bipolar I Disorder, Most Recent Episode Manic, In Full Remission; Bipolar I Disorder, Most Recent Episode Manic, In Partial Remission; Bipolar I Disorder, Most Recent Episode Manic, Mild; Bipolar I Disorder, Most Recent Episode Manic, Moderate; Bipolar I Disorder, Most Recent Episode Manic, Severe With Psychotic Features; Bipolar I Disorder, Most Recent Episode Manic, Severe Without Psychotic Features; Bipolar I Disorder, Most Recent Episode Manic, Unspecified; Bipolar I Disorder, Most Recent Episode Mixed, In Full Remission; Bipolar I Disorder, Most Recent Episode Mixed, In Partial Remission; Bipolar I Disorder, Most Recent Episode Mixed, Mild; Bipolar I Disorder, Most Recent Episode Mixed, Moderate; Bipolar I Disorder, Most Recent Episode Mixed, Severe With Psychotic Features; Bipolar I Disorder, Most Recent Episode Mixed, Severe Without Psychotic Features; Bipolar I Disorder, Most Recent Episode Mixed, Unspecified; Bipolar I Disorder, Most Recent Episode Unspecified; Bipolar I Disorder, Most Recent Episode Hypomanic; Bipolar I Disorder, Single Manic Episode, In Full Remission; Bipolar I Disorder, Single Manic Episode, In Partial Remission; Bipolar I Disorder, Single Manic Episode, Mild; Bipolar I Disorder, Single Manic Episode, Moderate; Bipolar I Disorder, Single Manic Episode, Severe With Psychotic Features; Bipolar I Disorder, Single Manic Episode, Severe Without Psychotic Features; Bipolar I Disorder, Single Manic Episode, Unspecified; Bipolar II Disorder; Body Dysmorphic Disorder; Borderline Personality Disorder; Breathing-Related Sleep Disorder; Brief Psychotic Disorder; Bulimia Nervosa; Circadian Rhythm Sleep Disorder; Conversion Disorder; Cyclothymic Disorder; Delusional Disorder; Dependent Personality Disorder; Depersonalization Disorder; Depressive Disorder NOS; Dissociative Amnesia; Dissociative Disorder NOS; Dissociative Fugue; Dissociative Identity Disorder; Dyspareunia; Dyssomnia NOS; Dyssomnia Related to (Another Disorder); Dysthymic Disorder; Eating Disorder NOS; Exhibitionism; Female Dyspareunia Due to Medical Condition; Female Hypoactive Sexual Desire Disorder Due to Medical Condition; Female Orgasmic Disorder; Female Sexual Arousal Disorder; Fetishism; Frotteurism; Gender Identity Disorder in Adolescents or Adults; Gender Identity Disorder in Children; Gender Identity Disorder NOS; Generalized Anxiety Disorder; Histrionic Personality Disorder; Hypoactive Sexual Desire Disorder; Hypochondriasis; Impulse -Control Disorder NOS; Insomnia Related to (Another Disorder); Intermittent Explosive Disorder; Kleptomania; Major Depressive Disorder, Recurrent, In Full Remission; Major Depressive Disorder, Recurrent, In Partial Remission; Major Depressive Disorder, Recurrent, Mild; Maj or Depressive Disorder, Recurrent, Moderate; Maj or Depressive Disorder, Recurrent, Severe With Psychotic Features; Major Depressive Disorder, Recurrent, Severe Without Psychotic Features; Major Depressive Disorder, Recurrent, Unspecified; Major Depressive Disorder, Single Episode, In Full Remission; Major Depressive Disorder, Single Episode, In Partial Remission; Major Depressive Disorder, Single Episode, Mild; Major Depressive Disorder, Single Episode, Moderate; Major Depressive Disorder, Single Episode, Severe With Psychotic Features; Major Depressive Disorder, Single Episode, Severe Without Psychotic Features; Major Depressive Disorder, Single Episode, Unspecified; Male Dyspareunia Due to Medical Condition; Male Erectile Disorder; Male Erectile Disorder Due to Medical Condition; Male Hypoactive Sexual Desire Disorder Due to Medical Condition; Male Orgasmic Disorder; Mood Disorder Due to Medical Condition; Narcissistic Personality Disorder; Narcolepsy; Nightmare Disorder; Obsessive Compulsive Disorder; Obsessive-Compulsive Personality Disorder; Other Female Sexual Dysfunction Due to Medical Condition; Other Male Sexual Dysfunction Due to Medical Condition; Pain Disorder Associated with both Psychological Factors and Medical Conditions; Pain Disorder Associated with Psychological Features; Panic Disorder with Agoraphobia; Panic Disorder without Agoraphobia; Paranoid Personality Disorder; Paraphilia, NOS; Parasomnia NOS; Pathological Gambling; Pedophilia; Personality Disorder NOS; Posttraumatic Stress Disorder; Premature Ejaculation; Primary Hypersomnia; Primary Insomnia; Psychotic Disorder Due to Medical Condition, with Delusions; Psychotic Disorder Due to Medical Condition, with Hallucinations; Psychotic Disorder, NOS; Pyromania; Schizoaffective Disorder; Schizoid Personality Disorder; Schizophrenia, Catatonic Type; Schizophrenia, Disorganized Type; Schizophrenia, Paranoid Type; Schizophrenia, Residual Type; Schizophrenia, Undifferentiated Type; Schizophreniform Disorder; Schizotypal Personality Disorder; Sexual Aversion Disorder; Sexual Disorder NOS; Sexual Dysfunction NOS; Sexual Masochism; Sexual Sadism; Shared Psychotic Disorder; Sleep Disorder Due to A Medical Condition, Hypersomnia Type; Sleep Disorder Due to A Medical Condition, Insomnia Type; Sleep Disorder Due to A Medical Condition, Mixed Type; Sleep Disorder Due to A Medical Condition, Parasomnia Type; Sleep Terror Disorder; Sleepwalking Disorder; Social Phobia; Somatization Disorder; Somatoform Disorder NOS; Specific Phobia; Transvestic Fetishism; Trichotillomania; Undifferentiated Somatoform Disorder; Vaginismus; and Voyeurism.

Preferably in the uses and methods of preventing or treating nerve cell death or damage or exerting neuroprotection or regenerating nerve cells by administering MTA, its pharmaceutically acceptable salts and/or prodrugs thereof, the subject suffers from a neurological disease (selected from multiple sclerosis, progressive multiple sclerosis, Parkinson's disease, Alzheimer's disease, neuromyelitis optica, neuroinflammation, amyotrophic lateral sclerosis (ALS), Friedreich's ataxia, Huntington's disease, Dementia with Lewy bodies, spinal muscular atrophy, brain ischemia, global brain ischemia, optic nerve ischemia, optic neuritis, brain tumor, brain trauma and epilepsy, encephalopathy, encephalitis, meningitis, chronic pain, migraine, headache, chronic fatigue and fibromialgia) or a psychiatric disease (selected from depression, bipolar disorder, schizophrenia, obsessive-compulsive disease, alcohol abuse, drug abuse ).

MTA, its pharmaceutically acceptable salts and/or prodrugs thereof may be used as a first line or initial monotherapy to prevent or treat nerve cell death or damage. Alternatively, MTA, its pharmaceutically acceptable salts and/or prodrugs thereof may be used as adjunct or as an add-on therapy to other drugs in a subj ect already being treated for a particular neurological or psychiatric disease, for example as an add-on to existing epilepsy treatment in patients who have partial seizures (epileptic fits starting in one specific part of the brain) It can be used in patients with and without secondary generalisation (where the seizure subsequently spreads to the whole brain). It can also be used in patients treated for brain ischemia, multiple sclerosis, neuromyelitis optica, Parkinson's disease, Alzheimer's disease, amyotrophic lateral sclerosis, Friedreich's ataxia, Huntington's disease, Dementia with Lewy bodies, or spinal muscular atrophy.

Thus, in another embodiment of the present invention, in the uses and methods of neuroprotection or of prevention or treatment of nerve cell death or damage or regeneration of nerve cells, MTA, its pharmaceutically acceptable salts and/or prodrugs thereof is used as adjunctive or add-on therapy to a subject with a neurological or psychiatric disease.

In one embodiment of the present invention, in the various uses and methods of neuroprotection or of prevention or treatment of nerve cell death or damage or regeneration of nerve cells, MTA, its pharmaceutically acceptable salts and/or prodrugs thereof is administered to a healthy subject, preferably a healthy subject older than 18 years old, more preferably a healthy subject older than 45 years old, even more preferably a healthy subject older than 55, 60, 65, 70, 75, 80, 85, 90, 95, or 100 years old.

The term "healthy subject" is meant to comprise its plain meaning as well as those subjects that may suffer from one or more pathological conditions other than a neurological or psychiatric disease.

In a further embodiment, in the various uses and methods of neuroprotection or of prevention or treatment of nerve cell death or damage or regeneration of nerve cells, MTA, its pharmaceutically acceptable salts and/or prodrugs thereof is used as adjunctive or add-on therapy to a subject being treated with one or more neuroenhancing drugs.

Neuroenhancing drugs include those that improve learning and memory, attention, mood, communicative skills and sexual performance. Examples of neuroenhancing drugs are those that target long-term synaptic potentiation (LTP) or long-term depression (LTD), modulation of calcium channels, or the cAMP response element-binding (CREB) protein. cAMP is the acronym for cyclic adenosine monophosphate. Particular examples of neuroenhancing drugs are phosphodiesterase inhibitors like rolipram; donepezil; agonists of the NMDA glutamate receptor like D-cycloserine; ampakines; modafinil; methylphenidate.

The neuroprotective properties of MTA, its pharmaceutically acceptable salts and/or prodrugs thereof have as a consequence the partial or full prevention or treatment of the various disorders in the nervous system functions caused by the neuronal cell death or damage. Therefore, the present invention further relates to the use of MTA, its pharmaceutically acceptable salts and/or prodrugs thereof as active ingredient in the manufacture of a medicament for the prevention or treatment of a neurological or psychiatric disease. In other words, the present invention also relates to MTA, its pharmaceutically acceptable salts and/or prodrugs thereof for use in the prevention or treatment of a neurological or psychiatric disease. Similarly, the present invention also relates to a method of prevention or treatment of a neurological or psychiatric disease comprising administering to a subject in need thereof an effective amount of MTA, its pharmaceutically acceptable salts and/or prodrugs thereof. The neurological or psychiatric disease disease may be any one from those listed above. Preferably, the neurological or psychiatric disease is selected from optic nerve ischemia, progressive multiple sclerosis, neuromyelitis optica, amyotrophic lateral sclerosis (ALS), Parkinson's disease, Alzheimer's disease, Friedreich's ataxia, Huntington's disease, Dementia with Lewy bodies, spinal muscular atrophy, epilepsy, optic neuritis, brain trauma, brain tumor, depression, bipolar disorder, schizophrenia, obsessive-compulsive disease, alcohol abuse, drug abuse, encephalopathy, encephalitis, meningitis, chronic fatigue, fibromialgia, chronic pain, migraine, and headache.

MTA, its pharmaceutically acceptable salts and/or prodrugs thereof may be formulated into various pharmaceutical forms for administration purposes. As appropriate compositions there may be cited all compositions usually employed for systemically administering drugs, for example any solid (e.g. tablets, capsules, granules, etc.) or liquid composition (e.g. solutions, suspensions, emulsions, etc). To prepare the pharmaceutical compositions of MTA, an effective amount of MTA, optionally in salt form or a prodrug, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, intrathecal, intravenous or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier usually comprises sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent or a suitable wetting agent, or both, optionally combined with suitable additives of any nature in minor proportions, which additives do not introduce a significant deleterious effect on the skin. A review of the different pharmaceutical forms for drug administration and their preparation may be found in the book "Tratado de Farmacia Galenica", de C. Faulí i Trillo, 10th Edition, 1993, Luzán 5, S.A. de Ediciones.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The compositions in accordance with this invention, including unit dosage forms, may contain the active ingredient in an amount that is in the range of about 0,1 % to 70%, or about 0,5% to 50%, or about 1 % to 25%, or about 5% to 20%, the remainder comprising the carrier, wherein the foregoing percentages are w/w versus the total weight of the composition or dosage form.

The dose of MTA, its pharmaceutically acceptable salts and/or prodrugs thereof to be administered depends on the individual case and, as customary, is to be adapted to the conditions of the individual case for an optimum effect. Thus it depends, of course, on the frequency of administration and on the potency and duration of action of the compound employed in each case for therapy or prophylaxis, but also on the nature and severity of the disease and symptoms, and on the sex, age, weight co-medication and individual responsiveness of the subject to be treated and on whether the therapy is acute or prophylactic. Doses may be adapted in function of weight and for paediatric applications. Daily doses may be administered q.d. or in multiple quantities such as b.i.d., t.i.d. or q.i.d.

The following examples are intended to illustrate the present invention and not to limit it thereto.

### Examples

### Example 1: Effect of MTA in a neuronal culture model of ischemia

### Summary

We explored the neuroprotective potential of MTA in a neuronal model of ischemia in which astrocytes and neurons were co-cultured.

Cultures: Astrocytes and neurons from the cerebral cortex of rats were used. Cells were deprived of oxygen and glucose and the protective effects of MTA measured.

### Introduction

### Neuron-astrocyte co-cultures

Primary neuron cultures were obtained from the cortical lobes of E18 Sprague-Dawley rat embryos according to previously described procedures (Cheung et al., 1998, Neuropharmacology 37:1419-1429). The cells were resuspended in B27 Neurobasal medium plus 10% FBS and then seeded in 24-well plates at 1-2 x 10⁵ cells per well onto a monolayer of astrocytes which were previously prepared as described earlier (Vallejo-Illarramendi et al., 2005, Glia 50:276-279). One day later, the medium was replaced by B27-supplemented Neurobasal medium and 10 % fetal bovine serum, and maintained at 37°C and 5% CO₂. Cultures were used 8-9 days after plating. Cultured neurons and astrocytes were identified using antibodies to microtubule associated protein-2 and glial fibrillary acidic protein, respectively.

### Cell toxicity and viability assays

Cell death and cell viability in neuron-astrocyte co-cultures was assayed after 24 h of exposure to MTA (10 µM to 1 mM) using lactate dehydrogenase and calcein-AM assays respectively followed by fluorimetry measurements (Synergy-HT). The results are expressed as percentage of viable cells or LDH release versus controls, as the mean ± SEM of at least four independent experiments performed in triplicate.

### Oxygen-glucose deprivation

Ischemic damage was induced by incubating cell cultures in a hyperbaric chamber for 1 hour in nitrogen-saturated buffer containing NaCl 130 mM, KC1 5,4 mM, CaC1₂ 0,1323 mM, NaHCO₃ 0,26 mM, MgCl₂ 0,8 mM, NaH₂PO₄ 1,18 mM, 10 mM sucrose as a substitute of glucose, and the glyocolytic blocker iodoacetate (20 or 50 µM). After the insult, cells were incubated in normoxia, glucose added and iodoacetate removed for an additional period of 24 hours. MTA or the NMDA receptor antagonist APV (50 µM) were added during ischemia to evaluate their neuroprotective activity. The cultures were assayed for cell viability and cell death as above. The results are expressed as the mean ± SEM of at least four independent experiments performed in triplicate.

### Treatment

Cell death and cell viability in neuron-astrocyte co-cultures was assayed after 24 h of exposure to MTA (10 µM to 1 mM) using lactate dehydrogenase and calcein-AM assays respectively followed by fluorimetry measurements (Synergy-HT).

### Material and methods

### Test components

### 5'-deoxy-5'-methylthioadenosine (MTA)

### Animals

| | |
|---|---|
| Specie | Sprague-Dawley rats |
| Sex | Female |
| Age | Pregnant adults 8-16 weeks for reproduction |
| Body weight range | E18 embryos and newborn animals were used |
| Supplier | Harlan |
| Identification method | Cultures labelled with permanent ink |
| Acclimatization | 7 days after arrival to the animal facility |
| Housing conditions | 1 female rat in ventilated cages |
| Diet | Lab-chow (Harlan) |
| Water | Tap water |
| Bedding | Woodcuttings |
| Dose(s) | Vehicle |
| | 10 µM-1 mM MTA |
| Volume(s) of administration | Diluted at least 1 to 100 |
| Injection time | n.a. |
| Route and frequency of systemic administration | added to culture |
| Duration of the treatment | 24 hours |
| Choice of the dose(s) and routes of administration | Doses were selected on the basis of earlier experiments |
| Number / group | 5-10 animals/group |
| Number of groups | 3-5 cultures per experimental set |
| Individual animal identification | n.a. |

### Doses and Groups

Two groups of cultures, each consisting of 8-14 rats were tested per disease model.

### Groups:

1. Vehicle
2. Treated: 10 µM to 1 mM

### Experimental procedures

1. MTA production: MTA was supplied by ENANTIA S.L. (Barcelona, Espana).
2. Data analysis: Statistical analysis was performed using Student t-test, two-tailed.

### Results

The addition of MTA (10 µM-1 mM) for 24 hours to cultures of neurons showed that the toxicity of this compound is very low (Figure 1), as demonstrated using assays for cell viability (calcein fluorescence in viable cells; left) or cell death (LDH release in dead cells; right). In turn, astrocytes were resistant and viable to MTA in the conditions assayed.

After learning that MTA was innocuous at the conditions employed, we explored its neuroprotective potential in simulated ischemia. Thus, ischemia was mimicked by removing oxygen and glucose (OGD) from the culture media and adding iodoacetate (a glycolytic blocker; IAA). Ischemia-induced damage was reduced by MTA in mixed cultures of neurons and astrocytes obtained from the cerebral cortex (Figure 2). Two ischemia conditions were assayed using IAA at 20 or 50 µM (left and right, respectively). In both conditions, MTA was highly protective. For comparison, APV, an antagonist of the NMDA receptor, was also assayed. MTA at 10 µM was even more protective than APV (50 µM).

### Conclusion

MTA protects from simulated ischemia in mixed cultures of neurons and astrocytes obtained from the cerebral cortex.

### Example 2: Effect of MTA in a rat model of global ischemia

### Summary

Because of the results obtained in a cellular model of ischemia with MTA, the study of its effects on animal models of ischemia was considered. These included a model of focal ischemia by occlusion of the middle cerebral artery (MCAO) and another of global ischemia (four-vessel occlusion model).

Animals: Adult young male Wistar rats (200-250 g) were used. After ischemia the animals were treated with MTA as described below.

### Introduction

### Surgery and ischemia

Before surgical procedures, animals were fasted overnight. The rats were subj ected to middle cerebral artery occlusion (MCAO) for 90 min using the intraluminal filament model as described previously (Rickhag et al, 2006; J Neurochem 96:14-29). Blood flow was monitored transcraneally with a doppler laser (Perimed). All physiologic parameters of animals included in the study were within normal ranges and included animals showing typical ischemia-induced behavioral deficits, rotational asymmetry, and dysfunctional limb placement. Rectal and body temperature was maintained at 37 °C during surgery and ischemia with a heating pad. Transient forebrain ischemia was induced by occlusion of the vertebral and common carotid arteries for 10 min according to the method described by Pulsinelli and Brierley (1979; Stroke 10, 267-272.). Criteria for forebrain ischemia were bilateral loss of the righting reflex, paw extension and mydriasis. Rectal and body temperature was maintained at 37 °C during surgery and ischemia with a heating pad. Animals that did not fully lose their righting reflexes or who developed seizures following carotid artery occlusion were excluded from the study.

### Treatment

The animals were treated with MTA (30 or 100 mg/kg i.p.), twice daily reconstituted in Tris 300mM, or placebo (Tris 300mM) starting at 30 min of the onset of ischemia.

### Material and methods

### Test components

### 5'-deoxy-5'-methylthioadenosine (MTA)

### Animals

| | |
|---|---|
| Specie | Wistar rats |
| Sex | Male |
| Age | 8-10 weeks |
| Body weight range | 200-250 g |
| Supplier | Harlan |
| Identification method | Tail labelling with permanent ink |
| Acclimatization | 7 days after arrival to the animal facility |
| Housing conditions | 2-3 rats in ventilated cages |
| Diet | Lab-chow (Harlan) |
| Water | Tap water |
| Bedding | Woodcuttings |
| | Vehicle |
| Dose(s) | 30-100 mg/kg MTA * i.p |
| | Tris 300mM |
| Volume(s) of administration | 0,5-2 ml/injection |
| Injection time | < 5 s |
| Route and frequency of systemic administration | Intraperitoneally (i.p) with 1 ml syringe twice every 24 hours |
| Duration of the treatment | 3-7 days |
| Choice of the dose(s) and routes of administration | Doses were selected on the basis of earlier experiments |
| Number / group | 8-14 rats |
| Number of groups | 2 per each model |
| Individual animal identification | Rats numbered |

### Doses and Groups

Two groups of animals, each consisting of 8-14 rats were tested per disease model. Groups:
1. Vehicle: Tris 300mM i.p
2. Treated: MTA i.p.

### Experimental procedures

1. MTA production: MTA was supplied by ENANTIA S.L. (Barcelona, Spain).
2. Data analysis: Statistical analysis was performed using Student t-test, two-tailed.

### Results

### Focal ischemia

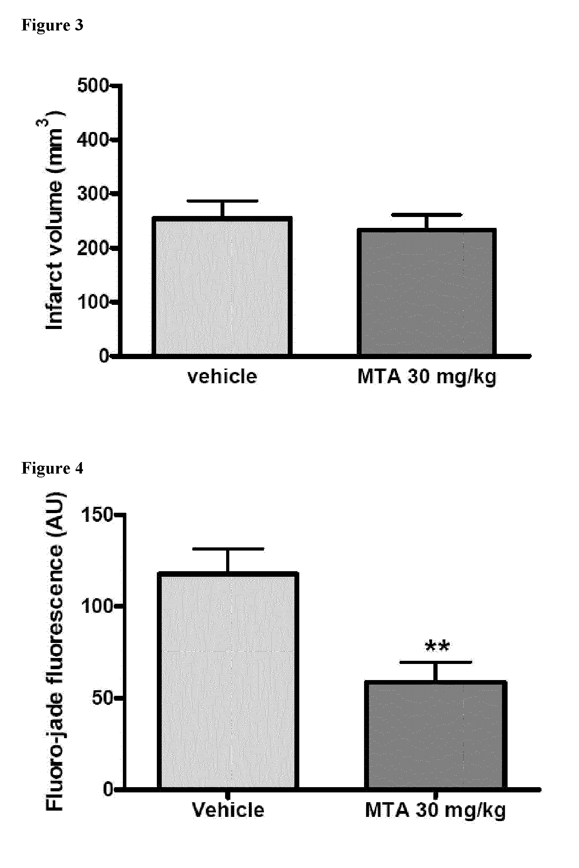

Occlusion of the middle cerebral artery (MCAO; 90 min) was followed by reperfusion for 3 days and MTA treatment (i.p. twice daily at 30 or 100 mg/kg) starting after 30 min of the onset of ischemia. Rats treated with MTA (30 mg/kg) did not show a reduction in the damaged area as compared to those treated with vehicle, as assessed with TTC staining (Figure 3). In turn, higher dose of MTA (100 mg/kg, twice daily) was toxic to rats after ischemia and could not be thoroughly evaluated.

### Global ischemia

Occlusion of vertebral and common carotid arteries (4-vessel occlusion model) for 10 min was followed by reperfusion for 7 days and MTA treatment (i.p. twice daily at 30 mg/kg), starting 20 min after recirculation (or 30 min after the onset of ischemia). Rats treated with MTA showed lower levels of dying cells (about 50 %; P<0.01) in the CA1 region of the hippocampal formation as compared to those animals treated with vehicle (Figure 4), as assessed with Fluoro-jade staining.

### Conclusion

MTA protects CA1 hippocampal neurons from cell death after 10 min global ischemia.

### Example 3: Effect of MTA in oligodendrocyte excitotoxicity and optic nerve ischemia

### Summary

We explored the neuroprotective potential of MTA in an oligodendrocyte model of excitotoxicity and a model of white matter ischemia.

Cultures: Oligodendrocytes were cultured from optic nerves from perinatal rats. Cells were exposed to excitotoxins and the protective effects of MTA measured.

Optic nerves: Whole optic nerves were isolated from young rats and subjected to experimental ischemia.

### Methods

### Oligodendrocyte cultures

Primary cultures of oligodendrocytes derived from optic nerves of 12-d-old Sprague Dawley rats were obtained as described previously (Barres et al., 1992). Cells were seeded into 24-well plates bearing 12-mm-diameter coverslips coated with poly-D-lysine (10 µg/ml) at a density of 5 x 10³ cells per well. Cells were maintained at 37 °C and 5% CO2 in a chemically defined medium (Barres et al., 1992, Cell 70:31- 46). After 2-4 d in vitro, cultures were composed of at least 98% 04/galactocerebroside-positive (O4/GalC_) cells; the majority of the remaining cells were stained with antibodies to glial fibrillary acidic protein (GFAP). No A2B5+ or microglial cells were detected in these cultures (Alberdi et al., 2002, Neurobiol Dis 9:234 -243).

### Cell viability and toxicity assays

Cell toxicity and viability assays were performed as described previously (Sánchez-Gómez and Matute, 1999, Neurobiol Dis 6:475-485). For evaluating the MTA toxicity, cells at 2-4 d in culture were exposed for 24 hours to that drug and cell viability assessed using calcein-AM (Invitrogen). For protection assays, cells were preincubated with MTA for 15 min and then exposed to AMPA for an additional period of 15 min. Twenty-four hours after drug application, cell viability was assessed using calcein-AM (Invitrogen). The total number of surviving cells on each coverslip emitting calcein fluorescence was counted, and results were expressed as percentage of cell death versus control. Results were expressed as the mean ± SEM of at least three independent experiments performed in duplicate.

### Ischemia in isolated optic nerves

Isolated adult optic nerves from Sprague Dawley rats were perfused with oxygen-saturated artificial CSF (aCSF) (in mM: 126 NaCl, 3 KCl, 2 MgSO₄, 26 NaHCO₃, 1.25 NaH₂PO₄, and 2 CaCl₂ 2H₂O, 10 glucose). Ischemic damage was induced by substituting glucose for sucrose and adding 1 mM of the glyocolytic blocker iodoacetate (1 mM) for 1 h followed by 2 h of reperfusion in nitrogen-saturated aCSF. Optic nerves during ischemia and reperfusion were incubated with vehicle, for controls, and with MTA or glutamate receptor antagonists, and subsequently processed for LDH release assay. The results are the mean ± SEM of at least three different experiments performed in duplicate.

### Treatment

Cell viability in oligodendrocyte cultures was assayed after 24 h of exposure to MTA (1 µM to 3 mM) calcein-AM assays respectively followed by fluorimetry measurements (Synergy-HT). Oligodendrocyte protection from excitotoxicity and optic nerve damage after ischemia (1 hour + 2 hours reperfusion) were examined using lactate dehydrogenase release assays in the presence or absence of MTA (100-300 µM for 3 hours).

### Test components

### 5'-deoxy-5'-methylthioadenosine (MTA)

### Animals

| | |
|---|---|
| Specie | Sprague-Dawley rats |
| Sex | Male/Female |
| Age | 12-day-old pups for culture; 8-10 week old rats for optic nerves |
| Body weight range | n.a. |
| Supplier | Harlan |
| Identification method | Cultures labelled with permanent ink |
| Acclimatization | 7 days after arrival to the animal facility |
| Housing conditions | 1 female rat and its off-springs in ventilated cages |
| Diet | Lab-chow (Harlan) |
| Water | Tap water |
| Bedding | Woodcuttings |
| Dose(s) | Vehicle |
| | 1 µM-3 mM MTA |
| Volume(s) of administration | Diluted at least 1 to 100 |
| Injection time | n.a. |
| Route and frequency of systemic administration | Added to cell cultures or optic nerves |
| Duration of the treatment | 24 h for cell cultures, 3 h for optic nerves |
| Choice of the dose(s) and routes of administration | Doses were selected on the basis of earlier experiments |
| Number / group | 5-10 animals/group for cultures, 2-3 animals/group for optic nerves |
| Number of groups | 3-5 cultures per experimental set, 6 optic nerves per experimental set |
| Individual animal identification | n.a. |

### Doses and Groups

Two groups of cultures or optic nerves.

### Groups:

1. Vehicle
2. Treated oligodendrocytes: 1 µM to 3 mM
Treated optic nerves: 100-300 µM

### Experimental procedures

1. MTA production: MTA was supplied by ENANTIA S.L. (Barcelona, Espana).
2. Data analysis: Statistical analysis was performed using Student t-test, two-tailed.

### Results

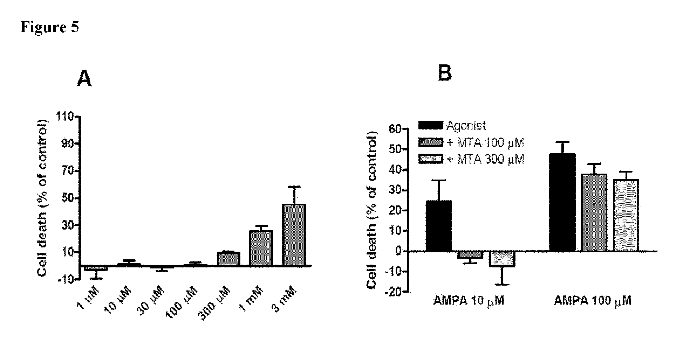

We first evaluated the toxicity profile of MTA (1 µM-3 mM) in oligodendrocyte cultures exposed to the compound for 24 hours, as assessed with calcein-AM fluorescence. The results indicated that significant toxicity was observed at MTA concentrations above 300 µM (Figure 5A). Then, we examined the potential protection of MTA of excitotoxic insults to oligodendrocytes induced by selective activation of ionotropic glutamate receptors of the AMPA type under experimental conditions in which MTA was not toxic to the cells (100-300 µM). At concentrations of the agonist inducing apoptosis or necrosis (10 µM of AMPA), MTA exerted a robust protective activity (Figure 5B). In contrast, no protection was observed under condition causing necrotic oligodendrocyte death (AMPA at 100 µM; Figure 5B).

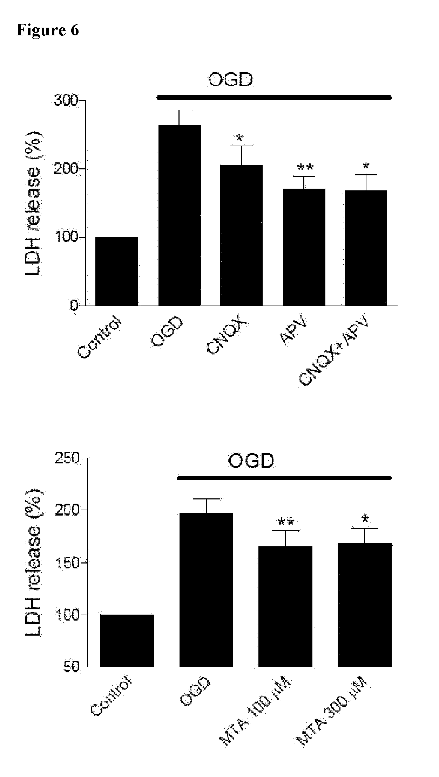

After learning that MTA was innocuous and protective at the conditions employed, we explored its ability to diminish white matter tissue damage in isolated optic nerves following simulated ischemia. Thus, ischemia was mimicked by removing oxygen and glucose (OGD) from the culture media and adding iodoacetate (a glycolytic blocker; IAA). Ischemia-induced damage was reduced by glutamate receptor antagonists of the AMPA and NMDA types using CNQX and APV respectively, alone or together (Figure 6, top). In addition, MTA was also similarly protective (Figure 6 bottom).

### Conclusion

MTA protects oligodendrocytes from excitotoxicity and optic nerves tissue damage after experimental ischemia.

### Example 4: Effect of MTA on excitoxic neuronal death

### Summary

The neuroprotective actions of MTA have been tested in a model of excitotoxicity (NMDA exposure). MTA does not show any neuroprotection against a neurotoxic insult in a pure neuronal culture. However, in a mixed glial-neuronal culture it showed a protective action as indicated by the blockade of caspase 3 activation. This suggests that glial cells might be required in the neuroprotective action of MTA in neuroexcitotoxicity.

### Introduction

The possible neuroprotective effect of MTA has been studied using a model of excitotoxicity-like exposure to the glutamate NMDA receptor agonist NMDA. The nNMDA neurotoxicity was studied in two models: 1) pure rat cortical neuronal culture and 2) mixed glial-cortical neuronal culture (Nicoletti et al., 1999, Neuropharmacology, 38:1477-1484).

### Material and methods

### Test components

### 5'-deoxy-5'-methylthioadenosine (MTA)

### Animals

| | |
|---|---|
| Specie | Sprague-Dawley rats |
| Sex | E17 embryos |
| Age | E17 |
| Body weight range | N/A |
| Supplier | Harlan |
| Identification method | N/A |
| Acclimatization | 7 days after arrival to the animal facility |
| Housing conditions | One pregnant mother in ventilated cages (57*35*19cm) |
| Diet | Lab-chow (Harlan) |
| Water | Tap water |
| Bedding | Woodcuttings |
| Dose(s) | N/A |
| Volume(s) of administration | N/A |
| Injection time | N/A |
| Route and frequency of systemic dministration | N/A |
| Duration of the treatment | N/A |
| Choice of the dose(s) and routes of administration | Doses were selected on the basis of earlier experiments |
| Number / group | Indicated in figure legends |
| Number of groups | Indicated in figure legends |
| Individual animal identification | N/A |

### Rat cortical neuron culture

Primary cultures of brain cortical neurons were essentially prepared as described previously (Bruno et al., 2001, Eur. J Neurosci., 13:1469-1478). The fronto-lateral cortical lobes were dissected out of Sprague-Dawley embryonic day 17 foetuses and were mechanically dissociated in Hanks' balanced solution. The cortical lobes were triturated by aspirating 7-10 times using a Pasteur pipette with a flame-narrowed tip. After centrifugation at 800g for 5 min, cells were resuspended in serum-free Neurobasal medium (GIBCO) supplemented with B27 (GIBCO) containing 2 mM L-glutamine, penicillin (20 units/ml) and streptomycin (5 µg/ml) and plated on poly-L-lysine-coated 24-well culture plates or on poly-L-lysine-coated 6-well culture plates. Cells were maintained at 37°C in a saturated humidity atmosphere containing 95% air and 5% CO₂ and cortical neurons were used for experiments after 7 days in vitro (DIV).

### Mixed rat cortical neuron and glia culture

Primary cultures of brain cortical neurons and glia were essentially prepared as described previously (Bruno et al., 2001, Eur. J Neurosci., 13:1469-1478). The fronto-lateral cortical lobes were dissected out of Sprague-Dawley embryonic day 17 foetuses and were mechanically dissociated in Hanks' balanced solution. The cortical lobes were triturated by aspirating 7-10 times using a Pasteur pipette with a flame-narrowed tip. After centrifugation at 800g for 5 min, cells were resuspended in serum-free Neurobasal medium (GIBCO) supplemented with B27 (GIBCO) and 5% fetal bovine serum containing 2 mM L-glutamine, penicillin (20 units/ml) and streptomycin (5 µg/ml) and plated on poly-L-lysine-coated 24-well culture plates or on poly-L-lysine-coated 6-well culture plates. Cells were maintained at 37°C in a saturated humidity atmosphere containing 95% air and 5% CO₂ and cortical neurons were used for experiments after 7 days in vitro (DIV).

### LDH assay

For viability experiments, cortical neurons or mixed cortical neurons and glia, seeded onto 24-well plates at 15 x 10⁴ cells/well and cultured for 7 DIV, were treated with vehicle (DMSO 1‰), NMDA (300 µM), NMDA (300 µM) + MTA (at different concentrations) or NMDA (300 µM) + MK-801 (10 µM) for 6 h and 24 h. Supernatants were collected and cells were washed with PBS and lysed with 0.9% Triton X-100 (v/v) in saline. Lactate dehydrogenase (LDH) activity was measured as an index of cellular death and mortality was expressed as percentage of LDH released to culture media. LDH was measured spectrophotometrically at 490 nm on a 96-well plate reader by using the Cytotox 96 Kit according to the manufacturer instructions (Promega ). The percentage of LDH release is defined by the ratio of LDH released over total LDH present in the cell at the beginning of treatment. All samples were run by triplicate (Posada et al., 2007, Br. J. Pharmacol., 150:577-585).

### MTT Assay

For viability experiments, cortical neurons seeded onto 24-well plates at 15 x 10⁴ cells/well and cultured for 7 DIV or SH-SY5Y neuroblastoma cells grown in 24-well culture plates until 80% confluence was reached were treated with vehicle (DMSO 1‰), NMDA (300 µM), NMDA (300 µM) + MTA (at different concentrations) or NMDA (300 µM) + MK-801 (10 µM) for 24 h. After incubation period, five mg/ml MTT were added to each well, being the volume of MTT added equal to one-tenth of the total volume of the well. This was followed by incubation at 37 °C for 3 h. After this, culture medium was removed and the insoluble formazan crystals were dissolved in 300 µl DMSO (Merck). Fifty microliter aliquots from each well were then transferred to a 96-well microplate, diluted with 150 µl DMSO and measured spectrophotometrically in an ELISA reader (Microplate Reader 2001, Bio-Whittaker) at reference wavelengths of 570 and 630 nm (Jordan et al., 2000, Br. J. Pharmacol., 130:1496-1504)

### Caspase 3 activity

Cortical neurons or mixed cortical neurons and glia were plated on poly-L-lysine-coated 6-well culture plates and after 7 DIV, cells were treated with vehicle or NMDA (300 µM) for different periods of time. In another set of experiments cells were treated with vehicle, NMDA alone or in the presence of MTA or MK-801 for 1 h. Afterwards, cells were washed twice with cold PBS and lysed in Lysis buffer containing 100 mM Hepes, 5 mM DTT, 5 mM EGTA, 0.04% Nonidet P-40, and 20% glycerol; pH 7.4. Extracts were then centrifuged at 5,000xg for 10 min at 4°C, and protein content was determined by using the BCA protein assay according to the manufacturer instructions. Cell extracts (40 µg of protein) were incubated in reaction buffer (25 mM Hepes, 10% sucrose, 0.1% CHAPS, 10 mM DTT) containing 50 µM fluorescence substrate Z-DEVD-AFC at 37°C for 1 h. Cleavage of the AFC fluorophore was determined in a spectrofluorometer at excitation wavelength of 400 nm and fluorescence was detected at an emission wavelength of 505 nm. Caspase 3 activity was expressed as units of fluorescence/mg of protein/h (Posadas et al., 2009, Pharm. Res., In press).

### Experimental procedures

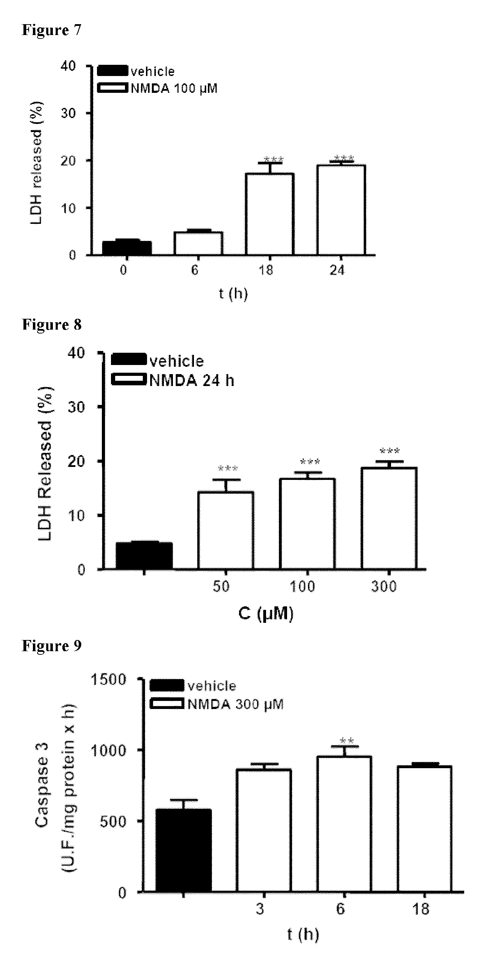

1. MTA production: MTA was supplied by ENANTIA S.L. (Barcelona, Spain)..
2. Data analysis: Data are expressed as mean ± s.e.m. Statistical analyses were carried out using the one-way analysis of variance (ANOVA) and the a posteriori Bonferroni's t-test for multiple comparisons. Statistics results are reported in the legends of figures 7-12. Statistical analysis was performed using Graph Pad Prism 4.0

### Results

Results are illustrated in Figures 7-12.

### Conclusions

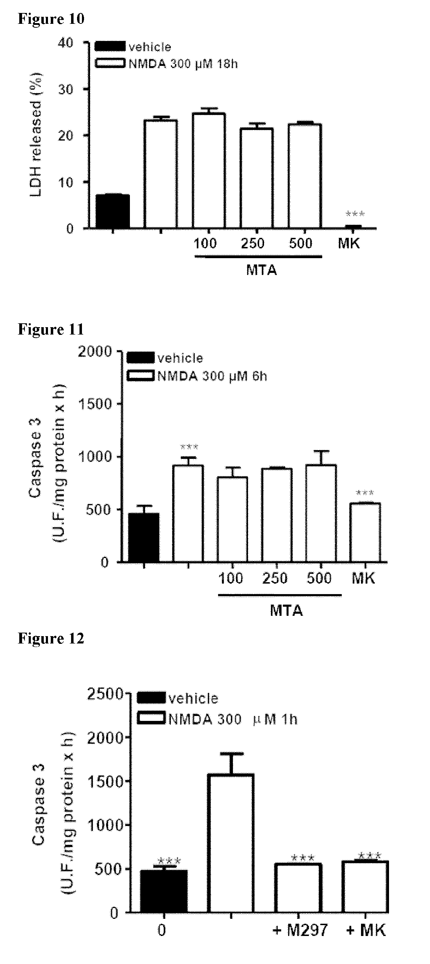

In the excitotoxicity model on rat cortical neurons, there is no neuroprotective effect of MTA up to 500 µM in pure neuronal cultures. The lack of effect has been observed on both LDH release and caspase 3 activity induced by the NMDA receptor agonist NMDA. However, when glial cells are present, protection against NMDA-induced caspase 3 activity can be observed (figure 12). This neuroprotective action is similar to the one observed using the NMDA receptor antagonist MK-801 (10 µM).

### Example 5: Evaluation of the neuroprotective activity of MTA in an in vitro model of neuroinflammation

### Summary

Neuroinflammation is a common process taking place in several neurological diseases such as Multiple Sclerosis, Alzheimer disease, Parkinson, ALS, or stroke, contributing to neurodegeneration. We have developed an in vitro model of neuroinflammation using organotypic cerebelar cultures stimulated with LPS for evaluating the neuroprotective activity of MTA. Results indicate that MTA decrease the number of dystrophic axons and prevented the loss of myelin, confirming the beneficial effect of MTA protecting axons and myelin against the immune mediated damage.

### Introduction

### Neuroinflammation model in cerebellar organotypic cultures.

Organotypic cultures model of neuroinflammation is based on previous observations in which systemic injections of lipopolysaccharide (LPS) in mice spinal cord induce inflammation, demyelinization and Wallerian degeneration. In mice cerebellar organotypic cultures, LPS induces microglia activation with consequent demyelination and axonal damage. In particular, LPS induces TNF-alpha, II,1-beta release, axonal swelling and end bulbs due to axonal transport block, and a decrease of axonal density. Organotypic cultures represent the best model to represent in vivo mechanisms since they maintain cell-cell interaction and organotypic structure of brain region studied. In this way, this in vitro neuroinflammation model reproduces the microglia activation, demyelinization and axonal damage phenomena observed in MS.

### Treatment

Cerebellar organotypic cultures which were pre-treated for half hour with 192 µM of MTA and 15 µg/ml of LPS have been used to generate neuroinflammation model.

### Material and methods

### Test components

### 5'-deoxy-5'-methylthioadenosine (MTA)

### Animals

| | |
|---|---|
| Specie | C57/B16 *wild type* mice |
| Sex | Male and Female |
| Age | 8-10 days |
| Body weight range | --- |
| Supplier | Harlan |
| Identification method | Plate labelling |
| Acclimatization | Animals were used 2 days after arrival to the animal facility |
| Housing conditions | 6 mice in ventilated cages with mother |
| Diet | Mother: Lab-chow (Harlan) |
| Water | Mother: Tap water |
| Bedding | Woodcuttings |
| | Vehicle |
| Dose(s) | 192µM MTA |
| | Tris 300mM |
| Volume(s) of administration | 57 ml/well |
| Injection time | < 5 s |
| Route and frequency of systemic administration | None (in vitro) |
| Duration of the treatment | 30 minutes |
| Choice of the dose(s) and routes of administration | Doses were selected on the basis of earlier experiments |
| Number / group | 5 mice P8-10 |
| Number of groups | 3 for each experiment (neuroinflammation model with 5 and 15 µg/ml LPS and model pre-treated with MTA) |
| Individual animal identification | mice numbered from 1 to 5 |

### Doses and Groups

Three groups of animals, each consisting of 5 mice P8-10.

### Group:

1. 15 µg/ml LPS in culture medium
2. 5 µg/ml LPS in culture medium
3. 192 µM MTA in culture medium plus 15 µg/ml LPS

### Experimental procedures

1. MTA production: MTA was supplied by ENANTIA S.L. (Barcelona, Spain).
2. Data analysis: Western blot, immunofluorescence and ELISA analysis were performed using conventional techniques.

### Results

Preliminary data from our laboratory have shown that the use of LPS in mouse cerebellar organotypic cultures produces the activation of microglia with the subsequent demyelinization and axonal damage in the cultures. Figures 13.I and 13.II show that the treatment with LPS induces the MHCII activation at three hours after the treatment (figure 13.II.B) and the release of TNF-alfa 1 hour after LPS-stimulation (figure 13.I.B). Figure 13.II.A shows inmunofluorescence images for MBP and NFL obtained with confocal microscopy. As can be shown, treating the organotypic cultures with LPS induces the demyelinization, not shown with the controls (figure 13.II.A panels 1 and 2). Also, an axonal swelling characteristic for an axonal blockade and knobs of axonal transection, as well as a reduction in the axonal density (Figure 13.II.A and panels 3 and 4) are observed.

In the in vitro neuroinflammation model, we observe significant differences between the cerebellar organotypic cultures treated with 15 µg/ml LPS and organotypic cultures pre-treated with MTA. In particular, immunofluorescent analysis for myelin basic protein (MBP) and neurofilaments light (NFL) shown demyelination and axonal damage in neuroinflammation model (Fig. 13), confirmed by Western blot analysis. Also, in this model we observe IL-β and TNF-α release (Fig. 13-14) suggesting microglia activation. Pretreatment for half hour with MTA before to induce neuroinflammation prevents demyelination and axonal damage, and decreases release of IL-β (Fig. 14). In summary, MTA is a neuroprotector in neuroinflammation model.

### Conclusion

MTA protects against demyelination a neurodegeneration in this model of neuroinflammation in organotypic cultures.

### Example 6: Neuroprotective activity of MTA in axonal damage in a transgenic model of progressive Multiple Sclerosis

### Summary

In Multiple Sclerosis, the presence of permanent disability is mainly dependent on axonal loss. For this reason, a critical end-point for disease modifying drugs for treating MS is whether they are able to prevent axonal loss due to the inflammatory process in the brain. MTA have shown cell protection properties in vitro and in animal models of liver diseases. In addition, we have shown that MTA has immunomodulatory properties and it is able to ameliorate the course and pathogenesis of the animal model of MS, Experimental Autoimmune Encephalomyelitis. Here we show that MTA prevent axonal loss in the EAE model, suggesting a neuroprotective activity which will be beneficial for treating the disease. In order to accurately measure axonal loss we made use of a transgenic mice that express fluorescent axonal in the motor pathway (the corticospinal tract).

### Methods

### Animal model of progressive MS

Chronic EAE was induced in Corticospinal tract-YFP mice (C57B6 background; see Bareyre et al., Nat Med. 2005 Dec; 11(12):1355-60) immunized with MOG. The fluorescence labelling of motor axons, allows an accurate quantification of axonal damage during EAE.

### Histological evaluation

1. Align spinal cords of all animals and cut out the following pieces of the spinal cord at the same level in all animals: a) a piece of the cervical spinal cord (about 1 cm in length) starting around at the upper cervical level around C1/2 (this will allow you to determine the number of CST fibers that enter the spinal cord); b) a piece of the lumbar spinal cord (about 1 cm in length) starting around the mid-lumbar level L2/3 (this will allow you to determine the number of fibers that reach the lumbar spinal cord).
2. Transfer spinal cord to 30% sucrose in PBS and wait for tissue to equilibrate (the spinal cord will sink to the bottom) - usually after 3-7 days.
3. Embed spinal cord pieces in tissue tek and carefully freeze (we use isopropanol cooled to about -40°C) - mark the direction of the spinal cord so you can be sure to start cutting at the upper (=cranial) end of the tissue.
4. Cut cryostat cross-sections starting from the upper end of the spinal cord piece (about 50 micrometer thick) and mount them on a adhesive glass slide (e.g SuperFrost) - we usually cut about 20 sections from the cervical and lumbar piece per animal / store the rest of the tissue block at -20 ° C.
5. Mount the tissue and analyze the sections using confocal microscopy - use a high-resolution objectives e.g. a 60x oil objective and take a single image plan covering the entire corticospinal tract.
6. Then simple count the number of dots (= number of fibers) - we usually do this blinded on 3 - 5 sections and then calculate the average for the CST as the CST - especially in the cervical spinal cord which contains many fibers it might be easier to estimate the total number of cervical CST fibers based on counts of several CST areas with a defined size. By counting the number of cervical and lumbar CST fibers in each animals you can determine the reduction of the CST. Further, if there is a difference between the groups, I can send you some spinal cords of healthy animals the next time so you can normalize the axonal reduction to the expected reduction in healthy animals (due to CST fibers terminating above the lumbar spinal cord) and get the percentage of CST fibers lost to EAE (usually around 40 % in our experiments).

### Material and methods

### Test components

### 5'-deoxy-5'-methylthioadenosine (MTA)

### Animals

| | |
|---|---|
| Specie | Corticospinal tract-YFP (C57B6 background) |
| Sex | Female |
| Age | 8-12 weeks |
| Body weight range | 20 grams |
| Supplier | Harlan |
| Identification method | Ear clipping tag |
| Acclimatization | 7 days after arrival to the animal facility |
| Housing conditions | 8 mice in ventilated cages |
| Diet | Lab-chow (Harlan) |
| Water | Tap water |
| Bedding | Woodcuttings |
| Dose(s) | Placebo (PBS) |
| | MTA 96 µmol/Kg |
| Volume(s) of administration | 200 µL |
| Injection time | < 5 s |
| Route and frequency of systemic administration | intraperitoneally (i.p) with 1 ml syringe once every 24 hours |
| Duration of the treatment | 30 days |
| Choice of the dose(s) and routes of administration | Doses were selected on the basis of earlier experiments |
| Number / group | 8 / 10 mice |
| Number of groups | 2 |
| Individual animal identification | mice numbered from 1 to 8 / 10 |

### Doses and Groups

### Two groups of animals.

### Groups:

1. Placebo (PBS) (8 animals)
2. MTA i.p. 96 µmol/Kg (10 animals)

### Experimental procedures

1. MTA production: MTA was supplied by ENANTIA S.L. (Barcelona, Spain).
2. Data analysis: Statistical analysis was performed using SPSS 13.0.

### Results

Animals were treated when EAE became chronic (first day with an EAE score of 2 or higher (> day 20th after immunization). Animals were randomized to be treated with either MTA (96 µMol/Kg) or placebo (PBS). MTA treated animals showed an amelioration of the clinical symptoms than placebo (Figure 15).

We performed a quantitative measurement of the axonal density and the lumbar and cervical spinal cord. We found that MTA treated animals have a significantly higher axonal density than placebo animals (Figure 16), indicating that treatment with MTA in the chronic phase of the disease was able to prevent long term axonal loss due to the autoimmune attack against the brain.

### Conclusion

Our results indicate that treatment with MTA in the chronic phase of the disease was able to prevent long term axonal loss due to the autoimmune attack against the brain.

### Example 7. Neuroprotective Effect of MTA in Experimental Temporal Lobe Epilepsy

### Summary

Temporal lobe epilepsy (TLE) is associated with substantial neuronal loss in the hippocampal formation. In rodent models of TLE, an episode of status epilepticus (SE) leads to a similar pattern of hippocampal neuronal death followed by the development of spontaneous recurrent seizures. Neuroprotective compounds have shown some promise in ameliorating SE-induced cell death, but they have been less impressive in preventing epileptogenesis. We undertook studies to examine the neuroprotective effects of methylthioadenosine (MTA) in the pilocarpine model of TLE. One group of rats received MTA for 5 days beginning 2 days before SE (pre-SE MTA) and another group was given MTA for 3 days beginning at the termination of SE (post-SE MTA), a more clinically relevant treatment regimen. Brain sections were examined for cell loss either 3-5 days (early timepoint) or 30 days (late timepoint) after SE. Mossy fiber sprouting was also examined by Timm stain at the late timepoint. One control group received vehicle and underwent SE, and a second control group received MTA and saline in place of pilocarpine. We found that MTA treatment beginning either before or after SE qualitatively reduced neuronal loss in the dentate hilus and hippocampal areas CA1 and CA3 at both early and late timepoints as assessed by Nissl stain and NeuN immunohistochemistry. Preliminary quantification of NeuN-immunoreactive cells in area CA1 at 30 days after SE showed significant neuroprotective effects in the pre-SE MTA group (p=0.02) and a trend toward neuroprotection in the post-SE MTA group (p=0.057). No qualitative difference in mossy fiber sprouting was observed in either of the groups. These findings suggest that MTA exerts neuroprotective properties in the setting of SE-induced neuronal death, and warrants further study to determine whether MTA exhibits anti-epileptogenic properties in experimental TLE.

### Introduction

Temporal lobe epilepsy (TLE) is a common and often pharmacoresistant form of epilepsy associated with significant morbidity due to recurrent seizures and associated memory dysfunction. Humans with TLE show substantial neuronal loss in the hippocampal formation that is thought to play a role in both the epilepsy and related cognitive dysfunction. The hippocampal pathology of TLE is recapitulated in rodent models in which adult animals are subjected to an episode of status epilepticus (SE) that leads after a latent period to spontaneous recurrent seizures. This experimental paradigm models the common clinical scenario in which a child experiences a complicated febrile seizure or febrile SE, and after a number of years goes on to develop TLE.

No antiepileptogenic treatments exist for TLE. All current therapies are symptomatic and directed at preventing recurrent seizures. Neuroprotective compounds have shown some promise in ameliorating SE-induced cell death in experimental TLE, but they have been less impressive in preventing epileptogenesis. Thus, an urgent need exists for novel therapies that will prevent the development of epilepsy after a brain insult. We therefore undertook studies to examine the neuroprotective effects of methylthioadenosine (MTA) in the pilocarpine model of TLE. Pilocarpine is a muscarinic cholinergic agonist that acts as a chemoconvulsant to produce SE. The pilocarpine model is a commonly used TLE model that consistently produces epileptic animals with patterns of cell loss and aberrant reorganization very similar to human TLE.

We compared two MTA treatment regimens to examine its neuroprotective effect on pilocarpine-induced SE in adult rats. One regimen involved 5 days of MTA treatment beginning beginning 2 days before SE (pre-SE MTA). The other regimen, a more clinically relevant protocol, consisted of MTA treatment for 3 days beginning at the termination of SE (post-SE MTA). Preliminary results suggest substantial neuroprotective effects with both MTA treatment regimens.

### Materials and Methods

### Test components

### 5'-deoxy-5'-methylthioadenosine (MTA)

### Animals

| | |
|---|---|
| Specie | Rat (Sprague-Dawley) |
| Sex | Male |
| Age | |
| Body weight range | 175-200 g |
| Supplier | Charles River |
| Identification method | |
| Acclimatization | 3 days after arrival to the animal facility |
| Housing conditions | 2-3 rats in ventilated cages (8" x 17" x 7") |
| Diet | Lab-chow |
| Water | Tap water |
| Bedding | |
| Dose(s) | |
| Volume(s) of administration | |
| Injection time | < 5 s |
| Route and frequency of systemic administration | intraperitoneally (i.p) with 1 ml syringe once every 24 hours |
| Duration of the treatment | 3 or 5 days |
| Choice of the dose(s) and routes of administration | Doses were selected on the basis of earlier experiments |
| Number / group | 4-6 rats except control without SE (2 rats only) |
| Number of groups | 5 |
| Individual animal identification | rats numbered from 1 to 25 |

### Doses and Groups

Five groups of animals, 4-6/group except the group without SE (2 rats only).

### Groups:

1. Tris 100 mM for control
2. MTA 96 µM/Kg/day

### Treatment of animals with 5'-methylthioadenonsine (MTA)

Young adult (175-200g), male Sprage-Dawley rats were divided into four MTA treatment groups, 9-11 rats per group. In group A, rats were subjected to pilocarpine-induced *status epilepticus* (SE). Following 90 minutes of continuous seizure activity, SE was stopped by a single administration of diazepam (10 mg/kg). The rats were injected either with vehicle or MTA once daily for 3 days. Rats were sacrificed at day 4 after SE, and their brains perfusion fixed, frozen and sectioned for histology. In group B, treatment of rats was the same as Group A except that the rats were sacrificed at day 30 after SE. In group C, rats were treated with MTA once daily for two days. On the third day, rats underwent pilocarpine-induced SE. The rats were continuously treated with MTA once daily for 3 more days; controls received placebo The rats were then anesthetized and sacrificed day 4 after SE. In group D, rats were treated the same way as Group C except that the rats were sacrificed on 30 days after SE.

### Histology

The sections were subjected to cresyl violet (Nissl) staining and NeuN immunostaining to examine cell loss. Timm staining to identify mossy fiber sprouting was performed on the 30-day survival group.

### Experimental procedures

1. MTA production: MTA was supplied by ENANTIA S.L. (Barcelona, Spain).
2. Data analysis: Statistical analysis was performed using SPSS 11.0.

### Summary of Results

Cresyl violet staining showed qualitatively more Nissl-stained cells in hippocampi of MTA treated rats as compared with vehicle, regardless of whether MTA treatment was started before or after SE, at both early and late timepoints. Results of NeuN immunostaining were similar, and examples at the 30-day timepoint are shown in Figure 17. Differences in cell survival were qualitatively apparent in the dentate hilus, and hippocampal area CA3 and CA1 pyramidal cell layers. Quantification of Nissl-stained cell counts in area CA1 is shown in Figure 18. The findings suggest that MTA treatment is neuroprotective in this epilepsy model.

### Conclusion

MTA exerts neuroprotective properties in the setting of SE-induced neuronal death.

### Example 8. Neuroprotective effect of MTA in the animal model of Parkinson disease

**Summary:** We tested the effect of methyltioadenosine in the survival of dopaminergic neurons (TH+ cells) in the substantia nigra of the brainstem in mice treated with MPTP toxic for inducing the model of Parkinson disease.

### Introduction

In Parkinson disease several neuronal populations degenerate leading to a chronic degenerative disease with movement disorders, cognitive abnormalities and vegetative symptoms. Among the neuronal populations damaged by the disease, the most prominent are dopaminergic neurons from the substantia nigra in the brainstem, impairing the functioning of the basal ganglia. In order to study the biological basis of the disease several animal models are used, being the most commonly used the intoxication with MPTP (1-methyl-4-phenyl-1,2,3,6-tetrahydropyridine). MPTP intoxication in humans and animals produce a disease highly reminiscent of Parkinson disease, both at the clinical and histological level. Currently, Parkinson disease lacks treatments aimed to modifying the course of the disease (disease modifying drugs), although symptomatic therapy is well developed. Due to its high frequency, health and social cost, developing new therapies for stopping the course of the disease is a high priority. In this sense, therapies are aimed to protect or regenerate the dopaminergic neurons, which are primarily targeted by the diseases.

### Methods

### 1. The MPTP mice model

Male C57B1 mice, with a body ranging in weight from 23 to 31 g were used in this study. MPTP (Sigma) dissolved in 0.9% NaCl was injected intraperitonealy at a dose of 30 mg/kg for 5 days. MTA was dissolved in purified water with 2% of DMSO at a final concentration of 6mg/ml and was inj ected intraperitonealy at a dose of 96 µmol/Kg or 192 µmol/Kg. The first day of the experiment MTA was injected 24 hours before the MPTP injection and during the following four days administration of MTA was 1 hour before the administration of MPTP. Mice were divided in four groups: (1) MPTP (n=6); (2) MPTP plus 96 µmol/Kg MTA (n=6); (3) MPTP plus 192 µmol/Kg MTA (n=9) and (4) Control group (n=2).

At the end of the experiment, animals were anesthetized with an overdose of 10 % chloral hydrate in distilled water and then transcardially perfused with saline Ringer's solution followed by 100 ml of a cold fixative solution containing 4% paraformaldehyde and 0,4% glutaraldehyde in 0.125 M PB, pH 7.4. After perfusion, the skull was opened and the brain removed was post-fixed overnight at 4° C in the same fixative solution and next stored in a cryoprotective solution containing 20% glycerine and 2% dimethylsulphoxide in 0.125 M PB, pH 7.4. Frozen coronal microtome sections (50 µm-thick) were obtained and collected in 0.125 M PB, pH 7.4, in 6 series of adjacent sections. Sections were stored at -80 °C until further processing.

### Immunohistochemistry

With a random start, every third sections through the entire SN were sampled. The sections were processed free-floating. Endogenous peroxidase activity was removed by incubating the sections in methanol with 1.25% H2O2 for 40 min. After blocking with 4% normal goat serum, 4% BSA and 0.05% TX-100 in 0.125 M PBS (40 min, room temperature) the sections were incubated with a mouse antiTH monoclonal antibody (Sigma) diluted 1:500 (overnight at room temperature) in the same blocking solution. Next they were incubated with a biotinylated goat antimouse IgG antibody (Jackson ImmunoResearch) diluted 1:300 in 0.5% normal goat serum, 2% BSA in 0.125 M PBS for 60 min at room temperature. Sections were incubated in a 1:4000 solution of peroxidase-conjugated ExtrAvidin® in 0.125 M PBS for 90 min at room temperature and immunoreactive structures were visualized after incubation in 0.05% 3,3'-diaminobenzidine (DAB) and 0.03% fresh H₂O₂ in 0.05 M Tris-HCl (pH 7.4). Finally sections were mounted on glass slides using a 2% solution of gelatine in 0.05M Tris-HCl. The following day they were counterstained with Nissl (Sigma), dehydrated, cleared in xylene, and coverslipped with DPX. Before peroxidise inhibition, incubation with primary and secondary antibodies, peroxidise-conjugated and DAB incubation and mounting on glass slide, sections were rinsed 3 times with 0.125 M PBS. Histological quantification was done using the CAST system from Olympus using the BX50 microscope and following the methods described by Oorschot (J Comp Neurol 366:580-599, 1996). We used Cavalieru method for calculating volumes,, using 50 µm thickness sections. Neurons were counted using the optic dissector every 3 sections at 150 µm distance and at x400. In all cases the error coefficient was below 10%.

### Results

We found that animals treated with MPTP had a decrease in the number of dopaminergic neurons of 40% compared to control animals. By contrast, animals treated with MTA 96 µM/day had a significantly lower neuronal loss compared to control animals (10%) and significantly higher number of dopaminergic neurons than MPTP treated animals (30% more) (Figure 19).

### Conclusions

Our results shows that MTA protect dopaminergic neurons against damage induced by MPTP, suggesting that MTA can become a neuroprotective therapy (disease modifying therapy) for Parkinson disease.

### Example 9. Capacity of MTA to induce neuronal differentiation in an in vitro model and study of the signalling pathways involved

### Abstract

We tested the effect of methylthioadenosine (MTA) on the induction of the differentiation of neural precursors in neurons, determined by the production of neurites, in a dopaminergic cell line from rat pheochromocitome (PC12), and studied the signalling pathways involved.

### Introduction

The PC12 cell line is used as a model system for differentiating neuronal cells. After stimulation with nerve growth factor (NGF) it stops growing, starts forming processes and expressing other neuronal markers such as the formation of synaptic vesicles with the appropriate stimulus. It is well known that the ras-MAPK, phospholipase C (PLC) and phosphatidylinositol (PI) 3-kinase signalling pathways become activated upon treatment with NGF. The neurite differentiation system in PC12 is therefore a suitable system for studying the neurite forming capacity of MTA and the signalling pathways involved.

### Methods

### PC12 culture

The PC12 cells were maintained in HAM's medium at 37°C, supplemented with 2.5% of fetal bovine serum, 15% of horse serum and penicillin/streptomycine. The neurite differentiation assays were conducted using 24-well plates treated with collagen in HAM's medium supplemented with 0.5% of fetal bovine serum and penicillin/streptomycine. NGF (100 ng/ml) or MTA at different concentrations (25 µM, 100µM and 2 mM) were added to the cultures and, after four days, the percentage of cells with neurites longer than twice cell body. In each experiment a minimum of 300 cells were randomly counted.

### Determination of intracellular phosphorylation

The phosphorylation of different intracellular proteins was detected using Luminex technology. After the neurite differentiation assay the PC12 cells were received in lysis buffer and tested using different antibodies: total Akt/PKB, total p38/SAPK, total STAT3, total ERK/MAPK, total P70 S6 Kinase, phosphorolyated Akt/PKB (Ser473), phosphorylated Akt/PKB (Thr 308), phosphorylated p38/SAPK (Thr180/Tyr182), phosphorilyated STAT 3 (Tyr705/Ser 727), phosphorylated ERK/MAPK (Thr185/Tyr187) and phosphorylated P70 S6 Kinase (Thr412).

### Results

The neurite differentiation results reveal the capacity of MTA to develop new processes in PC12 cells. The formation of new neurites depends on the concentration of MTA added to the medium, being 1 mM the most optimal concentration. The highest concentration tested (5 mM) does not affect neurite development (Figure 20 A and B). In the MTA-induced differentiation process in PC12 cells we can observe activation of the MAPK, p38/SAPK and STAT3 pathways, which suggests that the effect of MTA on neurite differentiation may not follow the same pathway as NGF (which requires the inhibition of STAT3 phosphorylation (Figure 21)).

### Conclusions

The results show that MTA is capable of promoting the differentiation of neurons in PC12 cells using an alternative pathway to that classically used by NGF in the same cell line.

### Example 10. "In vitro" neuroprotective capacity of MTA against oxidative stress.

### Abstract

We tested the neuroprotective capacity of MTA in the RN22 rat Schwannoma cell line against two types of oxidative stress: hydrogen peroxide and copper sulphate. Neuroprotection was measured as the capacity to protect against stress-induced death and the capacity to reduce the production of reactive oxygen species (ROS) directly related to oxidative stress production.

### Introduction

The RN22 cell line (rat Schwannoma) is used as system for studying the protection against stress with copper sulphate due to the fact that, in this cell line, NGF is capable of activating survival pathways through its p75 membrane receptor. After stress generation, those cells whereto NGF is added activate survival pathways through the p75 receptor which allow them to survive under these conditions. The stress survival system in RN22 is therefore a suitable system for studying the capacity of MTA to protect nervous system cells against different types of stress.

### Methods

### RN22 culture

RN22 cells were maintained at 37°C in DMEM medium supplemented with 10% of fetal bovine serum and penicillin/streptomycine. The assays against stress with copper sulphate were conducted using 24-well plates in DMEM medium without serum. After allowing cell adherence for three days, NGF (100 ng/ml) or MTA was added to different concentrations half an hour before the copper sulphate (150 µM). After twenty-four hours, cell viability was quantified by MTT assay and ROS production was quantified by fluorescence. Assays against stress with hydrogen peroxide were conducted in the same way as the foregoing, using 100 µM hydrogen peroxide as a stress-producing agent.

### MTT assay

We determined the quantity of MTT (Sigma) that is reduced to insoluble formazan. After separating the medium, the water-insoluble formazan is solubilised in DMSO and the dissolved material is measured in a spectrophotometer at 570 nm.

### ROS measurement

We determined the quantity of ROS produced by the cells after stress with and without treatment. 2',7'- dichlorodihydrofluorescein (DCFH) is generally used to measure oxidative stress in cells. The cells were incubated with DCFH-DA (10 µM) at 37°C for thirty minutes. After incubation, the cells were washed twice with PBS and lysed by adding 10 mM Tris-HCL buffer containing 0.5% of Tween 20. The homogenates were then centrifuged at 10,000 g for ten minutes to separate the cell residue and DVF fluorescence in the supernatant was measured using a fluorometer (excitation wavelength 492-495 nm, emission wavelength 517-527 nm)(Invitrogen).

### Results

In the assays conducted to test neuroprotection against stress with copper sulphate we can observe that MTA exerts a significant neuroprotective effect, even reaching the protection levels of the NGF control in most of the MTA concentrations tested (Figure 22). The effect of MTA depends on the concentration of the molecule, being high concentrations (mM range) those which generate the lowest response. This effect was confirmed with the antioxidant capacity of MTA to reduce ROS production in different time periods after stress induction (Figure 23).

In the assays conducted to test neuroprotection against stress with hydrogen peroxide we can observe that MTA is capable of maintaining cell viability throughout the range of concentrations tested (25 µM-3 mM)(Figure 24). As in the case of stress with copper sulphate, MTA is capable of reducing ROS production in different time periods after stress with hydrogen peroxide (Figure 25).

### Conclusions

The results demonstrate the neuroprotective capacity of MTA against different types of oxidative stress. In both cases, MTA maintains cell viability after stress and has an antioxidant effect, significantly reducing ROS production levels in Schwan cells.

## Claims

1. Use of 5'-methylthioadenosine (MTA), its pharmaceutically acceptable salts and/or prodrugs thereof as active ingredient in the manufacture of a medicament for the prevention or treatment of nerve cell death or damage.

2. Use of MTA, its pharmaceutically acceptable salts and/or prodrugs thereof as active ingredient in the manufacture of a neuroprotective medicament.

3. Use of MTA, its pharmaceutically acceptable salts and/or prodrugs thereof as active ingredient in the manufacture of a medicament for the regeneration of nerve cells.

4. MTA, its pharmaceutically acceptable salts and/or prodrugs thereof for use in the prevention or treatment of nerve cell death or damage.

5. MTA its pharmaceutically acceptable salts and/or prodrugs thereof for use as a neuroprotective drug.

6. MTA, its pharmaceutically acceptable salts and/or prodrugs thereof for use in the regeneration of nerve cells.

7. A method of prevention or treatment of nerve cell death or damage comprising administering to a subject in need thereof an effective amount of MTA or of one of its pharmaceutically acceptable salts and/or prodrugs thereof.

8. A method of neuroprotection comprising administering to a subj ect in need thereof an effective amount of MTA or of one of its pharmaceutically acceptable salts and/or prodrugs thereof.

9. A method of regenerating nerve cells comprising administering to a subject in need thereof an effective amount of MTA or of one of its pharmaceutically acceptable salts and/or prodrugs thereof.

10. The use of MTA, its pharmaceutically acceptable salts and/or prodrugs thereof according to any one of claims 1-3, or MTA, its pharmaceutically acceptable salts and/or prodrugs thereof for use according to any one of claims 4-6, or the methods according to any one of claims 7-9, wherein MTA is administered to a subject with a neurological or psychiatric disease.

11. The use of MTA, its pharmaceutically acceptable salts and/or prodrugs thereof according to any one of claims 1-3, or MTA, its pharmaceutically acceptable salts and/or prodrugs thereof for use according to any one of claims 4-6, or the methods according to any one of claims 7-9, wherein MTA is used as adjunctive or add-on therapy to a subject with a neurological or psychiatric disease.

12. The use of MTA, its pharmaceutically acceptable salts and/or prodrugs thereof according to any one of claims 1-3, or MTA, its pharmaceutically acceptable salts and/or prodrugs thereof for use according to any one of claims 4-6, or the methods according to any one of claims 7-9, wherein MTA is administered to a healthy subject.

13. The use of MTA, its pharmaceutically acceptable salts and/or prodrugs thereof according to any one of claims 1-3, or MTA, its pharmaceutically acceptable salts and/or prodrugs thereof for use according to any one of claims 4-6, or the methods according to any one of claims 7-9, wherein MTA is used as adjunctive or add-on therapy to a subject being treated with one or more neuroenhancing drugs.

14. Use of MTA, its pharmaceutically acceptable salts and/or prodrugs thereof as active ingredient in the manufacture of a medicament for the prevention or treatment of a neurological or psychiatric disease selected from optic nerve ischemia, progressive multiple sclerosis, neuromyelitis optica, amyotrophic lateral sclerosis (ALS), Parkinson's disease, Alzheimer's disease, Friedreich's ataxia, Huntington's disease, Dementia with Lewy bodies, spinal muscular atrophy, epilepsy, optic neuritis, brain trauma, brain tumor, depression, bipolar disorder, schizophrenia, obsessive-compulsive disease, alcohol abuse, drug abuse, encephalopathy, encephalitis, meningitis, chronic fatigue, fibromialgia, chronic pain, migraine, and headache.

15. MTA, its pharmaceutically acceptable salts and/or prodrugs thereof for use in the prevention or treatment of a neurological or psychiatric disease selected from optic nerve ischemia, progressive multiple sclerosis, neuromyelitis optica, amyotrophic lateral sclerosis (ALS), Parkinson's disease, Alzheimer's disease, Friedreich's ataxia, Huntington's disease, Dementia with Lewy bodies, spinal muscular atrophy, epilepsy, optic neuritis, brain trauma, brain tumor, depression, bipolar disorder, schizophrenia, obsessive-compulsive disease, alcohol abuse, drug abuse, encephalopathy, encephalitis, meningitis, chronic fatigue, fibromialgia, chronic pain, migraine, and headache.

16. A method of prevention or treatment of a neurological disease comprising administering to a subj ect in need thereof an effective amount of MTA, its pharmaceutically acceptable salts and/or prodrugs thereof, wherein the neurological or psychiatric disease is selected from optic nerve ischemia, progressive multiple sclerosis, neuromyelitis optica, amyotrophic lateral sclerosis (ALS), Parkinson's disease, Alzheimer's disease, Friedreich's ataxia, Huntington's disease, Dementia with Lewy bodies, spinal muscular atrophy, epilepsy, optic neuritis, brain trauma, brain tumor, depression, bipolar disorder, schizophrenia, obsessive-compulsive disease, alcohol abuse, drug abuse, encephalopathy, encephalitis, meningitis, chronic fatigue, fibromialgia, chronic pain, migraine, and headache.
